# EUROPEAN PATENT APPLICATION

(11) **EP 4 201 400 A1**
(43) Date of publication of application: **28.06.2023**
(21) Application number: 21857560.3
(22) Date of filing: 12.08.2021
(51) Int. Cl.: A61K 9/127, A61K 47/18, A61K 48/00, C07C 229/16, C07C 255/24, C07D 295/13, C07C 233/36

(54) **LIPID NANOPARTICLE**

(30) Priority: 20.08.2020 CN 202010841678
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN)
(74) Representative: Epping - Hermann - Fischer
(86) International application number: PCT/CN2021/112167
(87) International publication number: WO 2022/037465

(57) **Abstract**

The present invention relates to a lipid nanoparticle. The lipid nanoparticle is formed from a compound having the following structure (I) or a pharmaceutically acceptable salt, tautomer or stereoisomer thereof. The present invention also provides use of the lipid nanoparticle for delivering a therapeutic agent component. The present invention also relates to use of the lipid nanoparticle in the manufacture of a medicament.

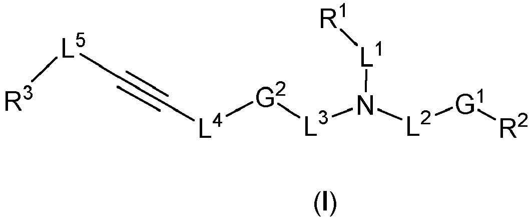

## Description

### Technical Field

The present invention relates to an amino lipid compound, a preparation process thereof, and a lipid particle containing the amino lipid compound. The lipid particle can be used to deliver a bioactive agent into a cell. The present invention also relates to use of the lipid particle containing the amino lipid as medicament.

### Background art

Gene therapy is to deliver genes with specific genetic information to target cells by artificial means, and the expressed target proteins have the effect of regulating, treating and even curing diseases caused by congenital or acquired gene defects. Both nucleic acid and cell membrane are negatively charged. Therefore, naked nucleic acids are difficult to be directly introduced into cells, and they are easily degraded by nucleic acid-degrading enzymes in the cytoplasm, which cannot achieve the effect of gene introduction and gene therapy. Therefore, it is necessary to use external force or vector to perform gene delivery.

Although the advantages of gene therapy are obvious, the lack of safety and high efficiency of gene vector prevents it from being widely used in clinic. Gene vectors are generally divided into viral vectors and non-viral vectors. The viral vectors have high transfection efficiency in vivo and in vitro, at the same time, they also have many defects, such as high toxicity, strong immune response, small gene capacity, poor targeting, and complicated preparation process. The non-viral vectors have attracted more and more attention due to their easy preparation, transportation, storage, safety, efficacy, and non-immunogenicity.

However, compared to gene delivery at the cellular level, gene delivery currently faces two problems during therapy. First, free RNA is prone to be digested by nucleases in plasma. A frequently used solution is to introduce phosphonates loaded with PEG chains in nanoparticles, and PEG chains stretch in the outermost layer of micelles during the self-assembly. Because it has the characteristics such as electric neutrality, protein adsorption resistance, and no functional group at the end group, the PEG layer can reduce the cytotoxicity of the nano-vector in vivo and prolong the cycle time. Second, after endocytosis, the gene vector will be transported to the endosome/lysosome vesicle, and the gene is easily degraded by enzymes or acidic substances rich in lysosomes. Therefore, whether nucleic acids can escape from endosomes/lysosomes and enter the cytoplasm is an important part of gene delivery by non-viral vectors. In the endosome/lysosome pathway, nano complexs will undergo a process of changing the acidity from the late endosomes at the pH of 5-6 to lysosomes at the pH of about 4.5. Meanwhile, lysosomes are rich in a large number of lysosomal enzymes, which can easily degrade nano complexes. Commonly used non-viral vectors have very low endosome/lysosome escape efficiency. Therefore, endosome/lysosome escape is a critical step to improve gene transfection from non-viral vectors. Disclosed herein is an alkynyl-containing amino lipid that concurrently contains multiple alkyl chains, which shows excellent ability to deliver nucleic acids into cells in the in vivo delivery study, and is significantly superior to the positive control (DLin-MC3) and the corresponding alkynyl-free amino lipid.

### Summary of the Invention

### Technical problems

One object of the present invention is to provide an amino lipid compound, which has an alkyl chain containing an alkynyl group, remain stable in the circulation in vivo, can be rapidly degraded in endosome/lysosome, and have significantly enhanced delivery efficiency.

Another object of the present invention is to provide a process for preparing the novel amino lipid compound with easily available raw materials, mild reaction conditions, good reaction selectivity, high yield, low requirements for equipment and apparatus and simple operation.

Another object of the present invention is to provide a lipid nanoparticle containing the amino lipid compound.

### Technical solutions

One aspect of the present invention provides a lipid nanoparticle, wherein the lipid nanoparticle contains a compound represented by formula I: 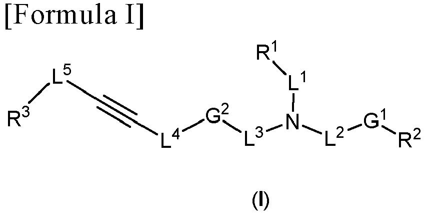 or a pharmaceutically acceptable salt, prodrug or stereoisomer thereof, wherein:
G¹ and G² are identical or different and each independently selected from -O(C=O)-, -(C=O)O-, -C(=O)-, -O-, -S(O)ₚ-, -S-S-, -C(=O)S-, -SC(=O)-, -NR^{a}C(=O)-, -C(=O)NR^{a}-, -NR^{a}C(=O)NR^{a}-, -OC(=O)NR^{a}- or -NR^{a}C(=O)O-; wherein p=0, 1, or 2; R^{a} is H or C₁-C₁₂ hydrocarbyl;
L¹, L², L³, L⁴ and L⁵ are identical to or different from each other, and each independently selected from C₁-C₂₄alkylene, C₂-C₂₄alkenylene, C₃-C₈cycloalkylene, C₃-C₈cycloalkenylene, or optionally substituted 4- to 10-membered heterocycle containing heteroatom(s) selected from nitrogen, sulphur, and oxygen, wherein, the C₁-C₂₄alkylene, the C₂-C₂₄alkenylene, the C₃-C₈cycloalkylene, and the C₃-C₈cycloalkenylene are optionally substituted by one or more substituent groups selected from hydrocarbyl, carboxyl, acyl, and alkoxy;
R² is a branched C₆-C₂₄alkyl or a branched C₆-C₂₄alkenyl;
R¹ and R³ are identical or different and each independently selected from H, OR^{1a}, CN, -C(=O)OR^{1a}, -OC(=O)R^{1a}, -NR^{1b}C(=O)R^{1a} or -NR^{1a}R^{1b}; wherein each of R^{1a} and R^{1b} is H or C₁-C₁₂ hydrocarbyl.

Another aspect of the present invention provides an amino lipid compound, wherein the amino lipid compound is a compound of formula I: 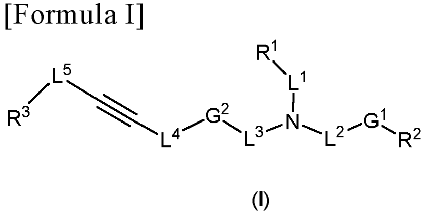 or a pharmaceutically acceptable salt, prodrug or stereoisomer thereof, wherein:
G¹ and G² are identical or different and each independently selected from -O(C=O)-, -(C=O)O-, -C(=O)-, -O-, -S(O)ₚ-, -S-S-, -C(=O)S-, -SC(=O)-, -NR^{a}C(=O)-, -C(=O)NR^{a}-, -NR^{a}C(=O)NR^{a}-, -OC(=O)NR^{a}- or -NR^{a}C(=O)O-; wherein p=0, 1, or 2; R^{a} is H or C₁-C₁₂ hydrocarbyl;
L¹, L², L³, L⁴ and L⁵ are identical to or different from each other, and each independently selected from C₁-C₂₄alkylene, C₂-C₂₄alkenylene, C₃-C₈cycloalkylene, C₃-C₈cycloalkenylene, or optionally substituted 4- to 10-membered heterocycle containing heteroatom(s) selected from nitrogen, sulphur, and oxygen, wherein, the C₁-C₂₄alkylene, the C₂-C₂₄alkenylene, the C₃-C₈cycloalkylene, and the C₃-C₈cycloalkenylene are optionally substituted by one or more substituent groups selected from hydrocarbyl, carboxyl, acyl, and alkoxy;
R² is a branched C₆-C₂₄alkyl or a branched C₆-C₂₄alkenyl;
R¹ and R³ are identical or different and each independently selected from H, OR^{1a}, CN, -C(=O)OR^{1a}, -OC(=O)R^{1a}, -NR^{1b}C(=O)R^{1a} or -NR^{1a}R^{1b}; wherein each of R^{1a} and R^{1b} is H or C₁-C₁₂ hydrocarbyl.

Preferably, the compound has the following structure: 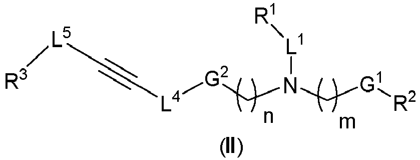 wherein m and n, identical or different, each independently is an integral number from 1 to 12.

Preferably, the compound has the following structure: 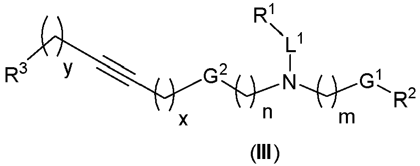 wherein x, y, m, and n, identical or different, each independently is an integral number from 1 to 12.

Preferably, the compound has one of the following structures (IVa) and (IVb): 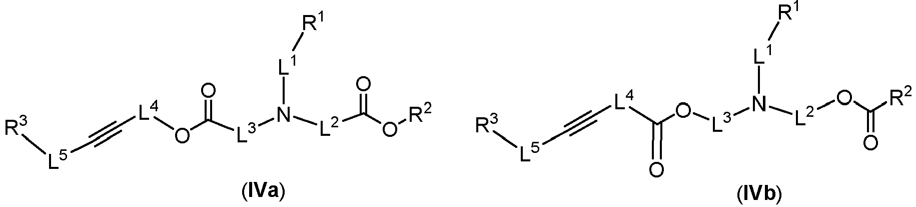 L¹, L², L³, L⁴, and L⁵ are identical or different and each independently selected from C₁-C₁₂alkylene, C₂-C₁₂alkenylene, C₃-C₈cycloalkylene, and C₃-C₈cycloalkenylene.

Preferably, the compound has one of the following structures (Va) and (Vb): 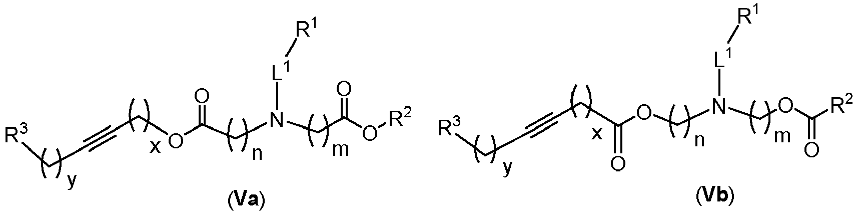 wherein x, y, m, and n, identical or different, each independently is an integral number from 1 to 12.

Preferably, R³ in the compound is H.

Preferably, R² in the compound is selected from those having the following structures:

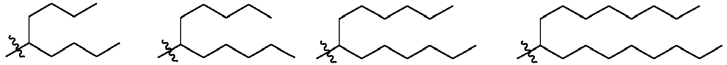

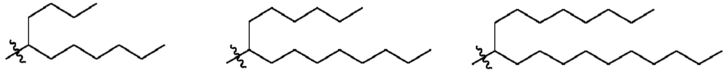

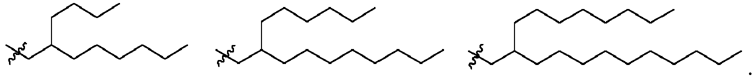

Further preferably, the compound of formula (I) is characterized in that,
G¹ and G² are identical or different and each independently selected from -O(C=O)- and -(C=O)O-;
L¹, L², L³, L⁴ and L⁵ are identical to or different from each other, and each independently is absent or represents C₁-C₁₃alkylene or C₄-C₆cycloalkyl;
R¹ and R³ are identical or different and each independently selected from H, OR^{1a}, CN, 4-6 membered saturated heterocyclyl containing one or two heteroatoms selected from N and O, -OC(=O)R^{1a}, -NR^{1b}C(=O)R^{1a} or -NR^{1a}R^{1b}; wherein each of R^{1a} and R^{1b} is H or C₁-C₁₂alkyl;R² is a branched C₆-C₂₄alkyl.

Further preferably, the compound of formula (I) is characterized in that,
G¹ and G² are identical or different and each independently selected from -O(C=O)- and -(C=O)O-;
L¹ is C₁-C₁₃alkylene or C₄-C₆ cycloalkyl; L², L³, L⁴ and L⁵ are identical to or different from each other, and each independently is absent or represents C₁-C₁₃alkylene;
R¹ is selected from OR^{1a}, CN, 4-6 membered saturated heterocyclyl containing one or two heteroatoms selected from N and O, -OC(=O)R^{1a}, -NR^{1b}C(=O)R^{1a} or -NR^{1a}R^{1b}; wherein each of R^{1a} and R^{1b} is H or C1-C₆ alkyl;R² is a branched C₆-C₂₄alkyl;
R³ is C1-C₆ alkyl.

Further preferably, the compound of formula (I) is characterized in that,
G¹ and G² are identical or different and each independently selected from -O(C=O)- and -(C=O)O-;
L¹ is C₁-C₆ alkylene or C₄-C₆ cycloalkyl;
L² is heptylene;
L³ is C₄-C₁₃alkylene;
L⁴ is absent or represents C₁-C₃alkylene;
L⁵ is C₂-C₈alkylene;
R¹ is selected from OH, CN, morpholinyl, -OC(=O)(CH₂)₄CH₃, -NHC(=O)(CH₂)₄CH₃ and-N(CH₃)₂;
R² is selected from 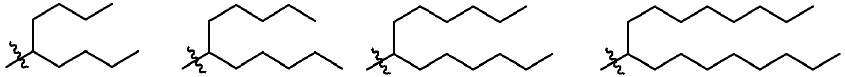 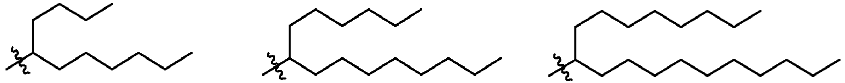 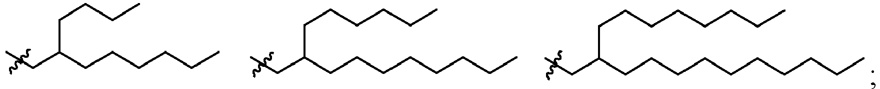
R³ is methyl.

The present invention provides the following representative compounds:

| No. | Structure |
|---|---|
| 1 | 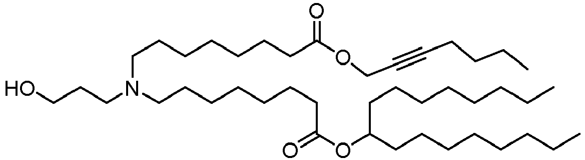 |
| 2 | 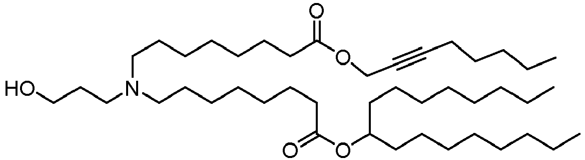 |
| 3 | 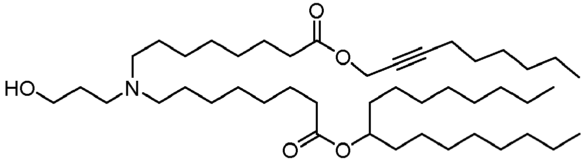 |
| 4 | 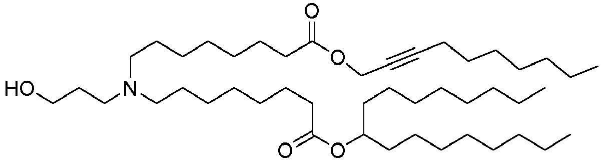 |
| 5 | 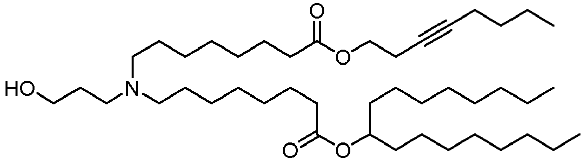 |
| 6 | 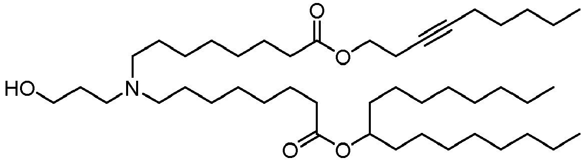 |
| 7 | 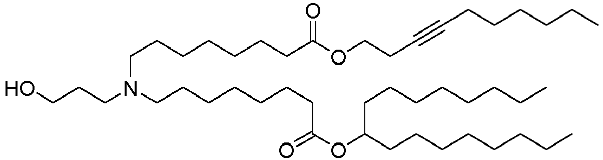 |
| 8 | 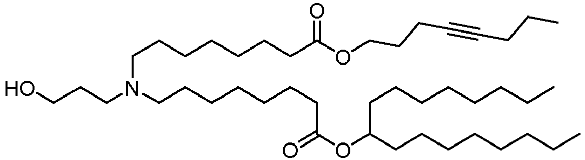 |
| 9 | 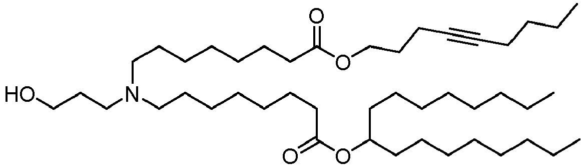 |
| 10 | 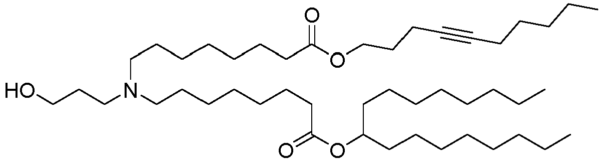 |
| 11 | 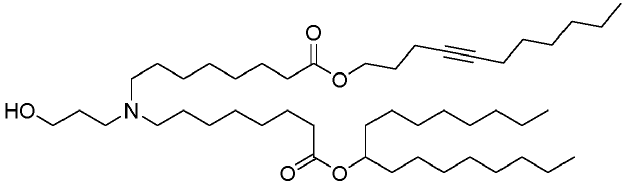 |
| 12 | 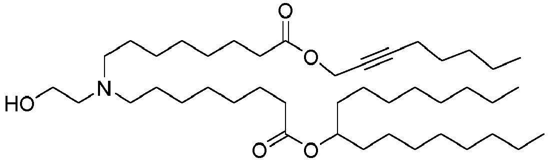 |
| 13 | 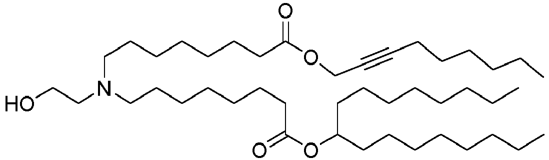 |
| 14 | 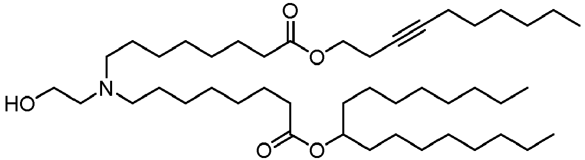 |
| 15 | 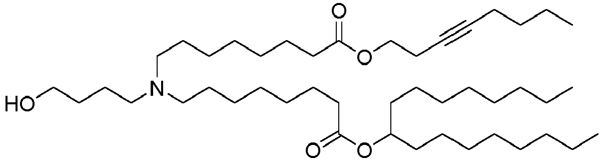 |
| 16 | 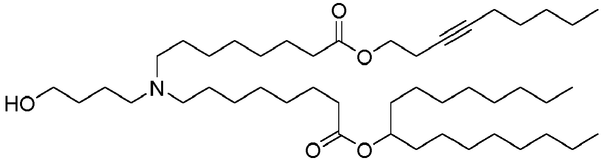 |
| 17 | 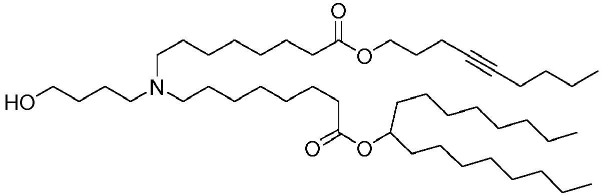 |
| 18 | 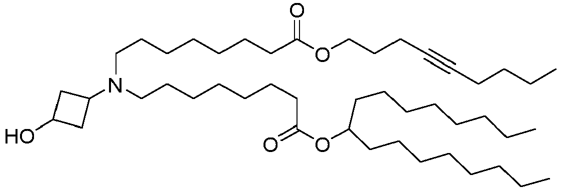 |
| 19 | 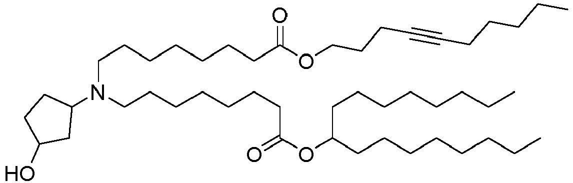 |
| 20 | 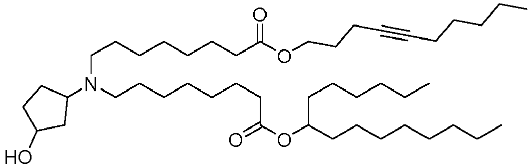 |
| 21 | 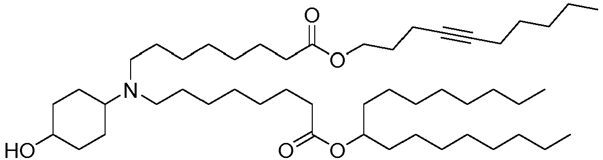 |
| 22 | 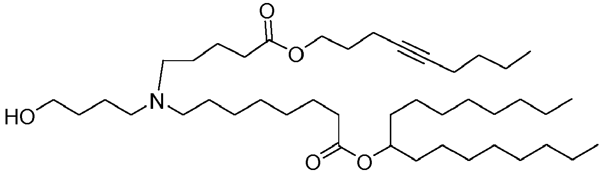 |
| 23 | 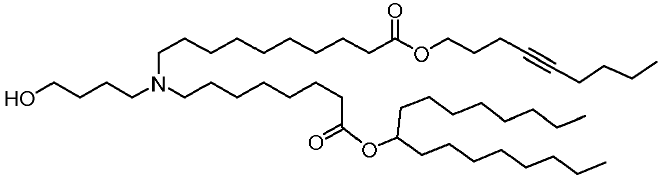 |
| 24 | 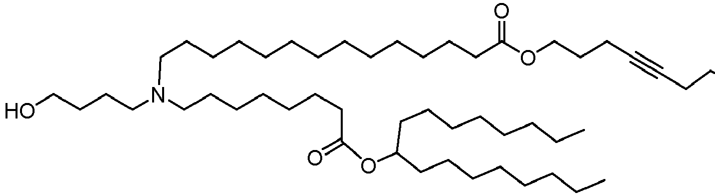 |
| 25 | 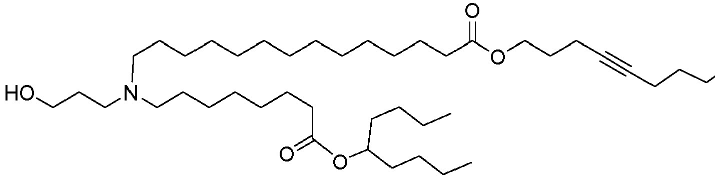 |
| 26 | 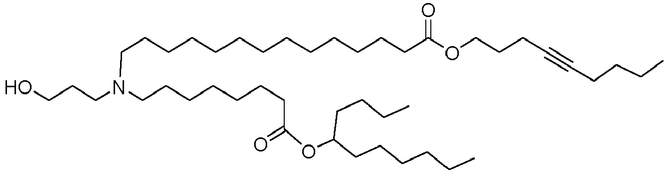 |
| 27 | 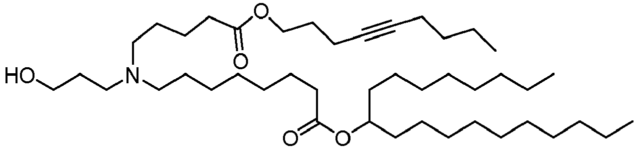 |
| 28 | 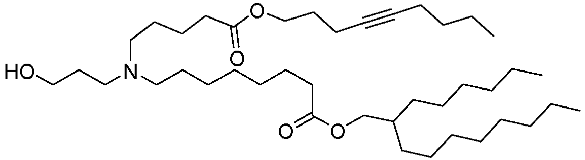 |
| 29 | 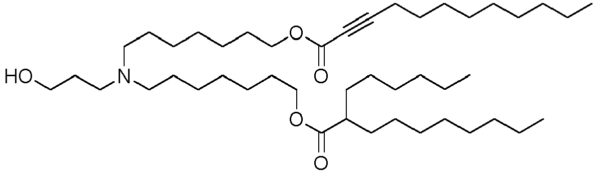 |
| 30 | 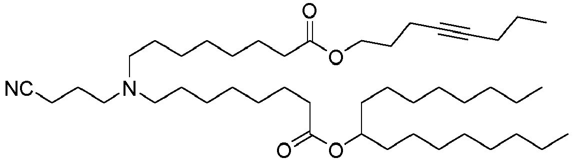 |
| 31 | 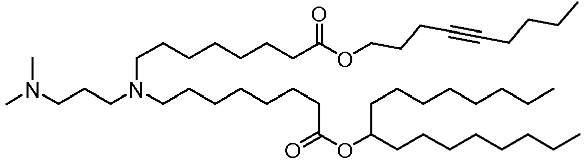 |
| 32 | 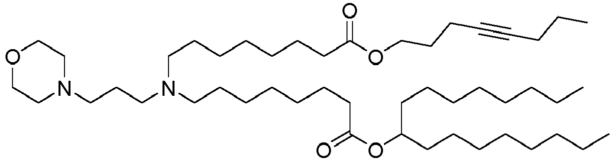 |
| 33 | 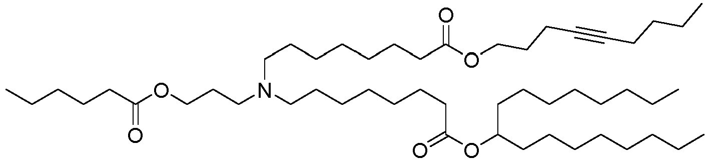 |
| 34 | 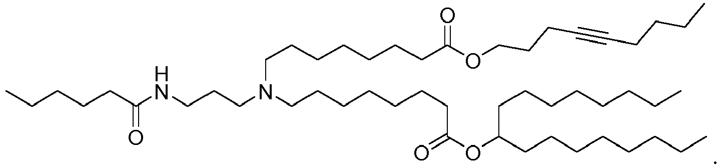 |

Another aspect of the present invention provides a process for preparing an amino lipid compound, which comprises the following steps:
(1) A compound represented by X-L²-G¹-R² is reacted with a compound represented by R¹-L¹-NH₂ in the presence of an organic base to produce a compound represented by R¹-L¹-NH-L²-G¹-R², wherein X is halogen; the organic base is preferably triethylamine, pyridine, DMAP, N,N-diisopropylethylamine; or,
   firstly a compound represented by HO-L²-G¹-R² undergoes oxidation reaction in the presence of Dess-Martin periodinane, then the achieved product is reacted with R¹-L¹-NH₂ in the presence of borohydride to produce a compound represented by R¹-L¹-NH-L²-G¹-R²; the borohydride is preferably sodium triacetoxyborohydride, sodium borohydride, potassium borohydride, sodium cyanoborohydride, or potassium cyanoborohydride;
(2) the compound represented by R¹-L¹-NH-L²-G¹-R² is reacted with 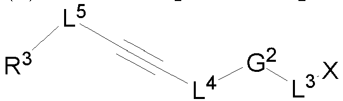 to produce the amino lipid compound, wherein X is halogen; preferably the reaction is performed in the presence of a base and a catalyst, the base is selected from an organic base or an inorganic base, the organic base is selected from triethylamine, pyridine, DMAP, and N,N-diisopropylethylamine, the inorganic base is selected from sodium carbonate, potassium carbonate, sodium bicarbonate, potassium bicarbonate, sodium hydroxide, potassium hydroxide, sodium hydride or potassium hydride, the catalyst is selected from iodine particles, sodium iodide or potassium iodide.

The present invention also provides a lipid particle containing the amino lipid compound and their use for delivering bioactive agents into cells. The present invention also comprises use of the lipid particle containing the amino lipid compound as medicaments.

### Beneficial Effect

The amino lipid compound of the present invention has an alkynyl group, and the addition of the alkyne lipid significantly increases the membrane fusion to enhance the mRNA release, resulting in the synergistic improvement in the mRNA delivery. It remains stable during the circulation in vivo and can be rapidly degraded in endosome/lysosome, with significantly enhanced delivery efficiency. The process for preparing the amino lipid compound has the merits such as easily available raw materials, mild reaction conditions, good reaction selectivity, high reaction yield, low requirements for equipment and apparatus and simple operation.

### Detailed description

Hereinafter, the present invention will be described in more detail.

The term "optionally substituted" as used herein means that one or more hydrogen atoms attached to an atom or group are independently unsubstituted, or are substituted by one or more substituents, e.g. one, two, three or four substituents, the substituents are independently selected from: deuterium(D), halogen, -OH, mercapto, cyano, -CD₃, C₁-C₆alkyl (preferably C₁-C₃alkyl), C₂-C₆alkenyl, C₂-C₆alkynyl, cycloalkyl (preferably C₃-C₈cycloalkyl), aryl, heterocyclyl (preferably 3- to 8-membered heterocyclyl), heteroaryl, arylC₁-C₆alkyl-, heteroarylC₁-C₆alkyl, C₁-C₆haloalkyl, -OC₁-C₆alkyl (preferably -OC₁-C₃alkyl), -OC₂-C₆alkenyl, OC₁-C₆alkylphenyl, C₁-C₆alkyl-OH (preferably C₁-C₄alkyl-OH), C₁-C₆alkyl-SH, C₁-C₆alkyl-O-C₁-C₆alkyl, OC₁-C₆haloalkyl, NH₂, C₁-C₆alkyl-NH₂ (preferably C₁-C₃alkyl-NH₂), -N(C₁-C₆alkyl)₂ (preferably -N(C₁-C₃alkyl)₂), -NH(C₁-C₆alkyl) (preferably -NH(C₁-C₃alkyl)), -N(C₁-C₆alkyl)(C₁-C₆alkylphenyl), -NH(C₁-C₆alkylphenyl), nitro, -C(O)-OH, -C(O)OC₁-C₆alkyl (preferably -C(O)OC₁-C₃alkyl), -CONRᵢRᵢᵢ (wherein Rᵢ and Rᵢᵢ are H, D and C₁-C₆alkyl, preferably C₁-C₃alkyl), -NHC(O)(C₁-C₆alkyl), -NHC(O)(phenyl), -N(C₁-C₆alkyl)C(O)(C₁-C₆alkyl), -N(C₁-C₆alkyl)C(O)(phenyl), -C(O)C₁-C₆alkyl, -C(O)heteroaryl (preferably -C(O)-5- to 7-membered heteroaryl), -C(O)C₁-C₆alkylphenyl, -C(O)C₁-C₆haloalkyl, -OC(O)C₁-C₆alkyl (preferably -OC(O)C₁-C₃alkyl), -S(O)₂-C₁-C₆alkyl, -S(O)-C₁-C₆alkyl, -S(O)₂-phenyl, -S(O)₂-C₁-C₆haloalkyl, -S(O)₂NH₂, -S(O)₂NH(C₁-C₆alkyl), -S(O)₂NH(phenyl), -NHS(O)₂(C₁-C₆alkyl), -NHS(O)₂(phenyl) and -NHS(O)₂(C₁-C₆haloalkyl), wherein each of said alkyl, cycloalkyl, phenyl, aryl, heterocyclyl and heteroaryl is optionally further substituted by one or more substituents selected from the following substituents: halogen, -OH, -NH₂, cycloalkyl, 3-to 8-membered heterocyclyl, C₁-C₄alkyl, C₁-C₄haloalkyl-, -OC₁-C₄alkyl, -C₁-C₄alkyl-OH, -C₁-C₄alkyl-O-C₁-C₄alkyl, -OC₁-C₄haloalkyl, cyano, nitro, -C(O)-OH, -C(O)OC₁-C₆alkyl, -CON(C₁-C₆alkyl)₂, -CONH(C₁-C₆alkyl), -CONH₂, -NHC(O)(C₁-C₆alkyl), -NH(C₁-C₆alkyl)C(O)(C₁-C₆alkyl), -SO₂(C₁-C₆alkyl), -SO₂(phenyl), -SO₂(C₁-C₆haloalkyl), -SO₂NH₂, -SO₂NH(C₁-C₆alkyl), -SO₂NH(phenyl), -NHSO₂(C₁-C₆alkyl), -NHSO₂(phenyl) and -NHSO₂(C₁-C₆haloalkyl). When one atom or group is substituted with a plurality of substituents, the plurality of substituents may be identical or different.

The term "hydrocarbyl" as used in the present invention means the group remained after an aliphatic hydrocarbon loses one hydrogen atom, including straight-chain or branched-chain, saturated or unsaturated hydrocarbon groups, including alkyl, alkenyl and alkynyl; preferably, the hydrocarbyl group is C₁-C₁₀hydrocarbyl, C₁-C₆hydrocarbyl, or C₁-C₃hydrocarbyl.

The term "alkyl" as used in the present invention refers to C₁-C₂₄alkyl, C₁-C₂₀alkyl, C₁-C₁₈alkyl, C₁-C₁₂alkyl, C₁-C₆alkyl, C₃-C₂₄alkyl, C₃-C₂₀alkyl, C₃-C₁₈alkyl, C₃-C₁₂alkyl, C₃-C₆alkyl, C₆-C₂₄alkyl, C₆-C₂₀alkyl, C₆-C₁₈alkyl or C₆-C₁₂alkyl.

The term "alkenyl" as used in the present invention refers to C₂-C₂₄alkenyl, C₂-C₂₀alkenyl, C₂-C₁₈alkenyl, C₂-C₁₂alkenyl, C₂-C₆alkenyl, C₃-C₂₀alkenyl, C₃-C₁₈alkenyl, C₃-C₁₂alkenyl, C₃-C₆alkenyl, C₆-C₂₄alkenyl, C₆-C₂₀alkenyl, C₆-C₁₈alkenyl or C₆-C₁₂alkenyl.

The term "alkynyl" as used in the present invention refers to C₂-C₂₄alkynyl, C₂-C₂₀alkynyl, C₂-C₁₈alkynyl, C₂-C₁₂alkynyl, C₂-C₆alkynyl, C₃-C₂₀alkynyl, C₃-C₁₈alkynyl, C₃-C₁₂alkynyl, C₃-C₆alkynyl, C₆-C₂₄alkynyl, C₆-C₂₀alkynyl, C₆-C₁₈alkynyl or C₆-C₁₂alkynyl.

The term "acyl" as used in the present invention refers to a hydrocarbyl-carbonyl group, preferably the acyl group is C₄-C₂₄acyl, C₆-C₁₈acyl, C₆-C₁₂acyl, C₆-C₁₀acyl, C₄-C₆acyl, C₂-C₁₂acyl, or C₂-C₆acyl.

The term "alkoxy" as used in the present invention refers to an alkyl-oxy group, preferably the alkoxy is C₁-C₁₀alkoxy, more preferably, the alkoxy is C₁-C₆alkoxy, most preferably, the alkoxy is C₁-C₃alkoxy.

The term "heterocycle" as used in the present invention refers to a saturated or unsaturated cyclic group containing heteroatom(s) selected from N, O, S, and the like, preferably the heterocycle is an optionally substituted 4- to 10-membered heterocycle containing 1-6 heteroatoms selected from N, O, and S, or an optionally substituted 4- to 6-membered saturated heterocycle containing 1, 2 or 3 heteroatoms selected from N, O, and S. The heterocycle may be optionally substituted with one or more substituents, as defined above for "optionally substituted".

Another embodiment of the present invention relates to a lipid particle containing the aforementioned amino lipid compound. The compounds of the present invention all have both a hydrophobic character due to their long non-polar residues and a hydrophilic character due to their amino group. This amphiphilic character can be used to form lipid particles, e.g., lipid bilayers, micelles, liposomes, and the like.

Within the scope of the invention, the term "lipid particle" means nanosized objects ( lipid nanoparticles) made of amino lipid compounds in an aqueous solution. These particles are inter alia lipid bilayer vesicles (liposomes), multi-lamellar vesicles or micelles.

In a preferred embodiment of the present invention, said lipid particles are liposomes containing the aforementioned amino lipid compounds. Within the scope of the invention, liposomes are microvesicles composed of a bilayer of lipid amphipathic (amphiphilic) molecules enclosing an aqueous compartment.

Liposome formation is not a spontaneous process. Lipid vesicles are formed first when lipids are placed in water and consequently form one bilayer or a series of bilayers, each separated by water molecules. Liposomes can be created by sonicating lipid vesicles in water.

Within the scope of the invention, the term "lipid bilayer" means a thin membrane made of two layers of lipid molecules. The term "micelle" means an aggregate of surfactant molecules dispersed in a liquid colloid. A typical micelle in aqueous solution forms an aggregate with the hydrophilic head regions upon contacting with water, chelating the hydrophobic single tail region in the micelle center.

Within the scope of the invention, the term "cells" means a generic term and encompass the cultivation of individual cells, tissues, organs, insect cells, avian cells, fish cells, amphibian cells, mammalian cells, primary cells, continuous cell lines, stem cells and/or genetically engineered cells, such as recombinant cells expressing a hetereologous polypeptide or protein. Recombinant cells include, for example, cells expressing heterologous polypeptides or proteins, such as a growth factor or a blood factor.

In a preferred embodiment, said lipid particles or liposomes further contain a helper lipid. In a preferred embodiment said helper lipid is a non-cationic lipid. In a more preferred embodiment said helper lipid is a non-cationic phospholipid. Within the scope of this invention, a non-cationic lipid may contain cationic functional groups (e.g. ammonium groups) but it should contain anionic functional groups to at least neutralize the molecule. The entirety of all functional groups in the lipid molecule should be non-cationic. Liposomes consisting of a mixture of cationic amino lipids and non-cationic (neutral) phospholipids are the most effective for nucleic acid delivery into cells. In an even more preferred embodiment said non-cationic lipid is DOPE or DSPC.

In a further preferred embodiment, the lipid particle or liposome further comprises a sterol. Sterol, like cholesterol, is a natural component in cell membranes. It can be used to stabilize the particle, and help the integration with cell membrane.

In another embodiment of the invention, the lipid particles or liposomes further contain a bioactive agent. Within the scope of this invention a bioactive agent is one which has a biological effect when introduced into a cell or host, for example , by stimulating an immune response or an inflammatory response, by exerting enzymatic activity or by complementing a mutation, etc. bioactive agents include inter alia nucleic acids, peptides, proteins, antibodies and small molecules. When a liposome is used to encapsulate a drug substance either within the lipid bilayer or in the interior aqueous space of the liposome, the term "liposome drug" can be employed.

In a most preferred embodiment, the bioactive agent is a nucleic acid. In another preferred embodiment said bioactive agent is a member selected from the group consisting of an antineoplastic agent, an antibiotic, an immunomodulator, an anti-inflammatory agent, an agent acting on the central nervous system, a polypeptide or a polypeptoid.

In yet another embodiment, the lipid particle or liposome further contains at least one polyethylene glycol (PEG)-lipid. PEG lipids help to protect the particles and their cargo from degradation in vitro and in vivo. Moreover, PEG forms a protective layer over the liposome surface and increase the circulating time in vivo. It can be used in liposome drug delivery (PEG-liposome). Preferably, the polyethylene glycol lipid is PEG2000-DMG. Lipid particles or liposomes containing a bioactive agent can be used to deliver any of a variety of therapeutic agents into cells. The present invention encompasses the use of lipid particles, especially liposomes, as described above for delivering a bioactive agent into a cell.

Preferably, said bioactive agent is a nucleic acid, including but not limited to, messenger RNA (mRNA), antisense oligonucleotide, DNA, plasmid, ribosomal RNA (rRNA), micro RNA (miRNA), transfer RNA (tRNA), small inhibitory RNA (siRNA) and small nuclear RNA (snRNA). The bioactive agent may also be an antineoplastic agent, an antibiotic, an immunomodulator, an anti-inflammatory agent, an agent acting on the central nervous system, an antigen or a fragment thereof, a protein, a peptide, a polypeptoid, a vaccine and a small-molecule or a mixture thereof. As shown above, lipid particles or liposomes containing amino lipid compounds as defined in the present invention are suitable to deliver bioactive agents into cells. The wide variety of different amino lipid compounds which can be synthesized by the mentioned general synthetic method can be screened for particular characteristics that are conferred to the liposomes, important characteristics are for example transfection efficiency, cytotoxicity, adhesion of the agent to be delivered into the cell, stability of the liposomes, size of the liposomes, etc. The present method allows the creation of specifically adapted liposomes for particular applications.

For example, lipid particles or liposomes can be used for transfecting multicellular tissues or organisms. This offers the possibility of a novel therapeutic treatment of patients.

According to the present invention, a patient can be any mammal, preferably selected from the group consisting of human, mouse, rat, pig, cat, dog, horse, goat, cattle, and monkey and/or others. Most preferably, the patient is a human being.

An important embodiment of the present invention is the use of said lipid particles or liposomes containing amino lipid compounds according to one of formulae (I-III) as medicament.

In particular, said lipid particles or liposomes can be administered to patients for use in gene therapy, in gene vaccination, in antisense therapy or in therapy by interfering RNA. Specific applications include but are not limited to:
(1) The lipid particles of the present invention can deliver nucleic acids for gene therapy. The exogenous gene is introduced into the target cell through the amino lipid of the present invention to correct or retrieve the disease caused by defects and abnormal genes, so as to achieve the purpose of treatment. It also includes the technical application of transgenosis and the like, namely, the exogenous gene is inserted into a proper receptor cell of a patient through a gene transfer technology, so that a product produced by the exogenous gene can treat certain diseases, such as common lung cancer, gastric cancer, liver cancer, esophagus cancer, colon cancer, pancreatic cancer, brain cancer, lymph cancer, blood cancer, prostate cancer and the like. Gene-edited nucleic acid substances can also be introduced for the treatment of various genetic diseases, such as hemophilia, Mediterranean anemia, Gaucher's disease, and the like.
(2) The lipid particles of the present invention can be used in vaccination. The lipid particle or liposome of the present invention may be used to deliver an antigen or a nucleic acid encoding an antigen. The lipid particle of the present invention may also be used to elicit an immune response against a wide variety of antigens for the treatment and/or prevention of a number of conditions including, but not limited to, cancer, allergy, toxicity and infection by pathogens such as viruses, bacteria, fungi, and other pathogenic organisms.

The above-mentioned lipid particles can be used to prepare a drug for nucleic acid transfer, preferably, the nucleic acid is RNA, messenger RNA (mRNA), antisense oligonucleotide, DNA, plasmid, ribosomal RNA (rRNA), microRNA (miRNA), transfer RNA (tRNA), small inhibitory RNA (siRNA), and small nuclear RNA (snRNA).

In order to make the purposes, technical solutions and advantages of the embodiments of the present invention clearer, the technical solutions in the embodiments of the present invention will be clearly and completely described below with reference to specific examples. Obviously, the described examples are a part of rather than all of the embodiments of the present invention. Based on the embodiments in the present invention, all other embodiments obtained by those of ordinary skill in the art without creative efforts shall fall within the protection scope of the present invention.

### Example 1: Synthesis of 1-octylnonyl 8-bromooctanoate

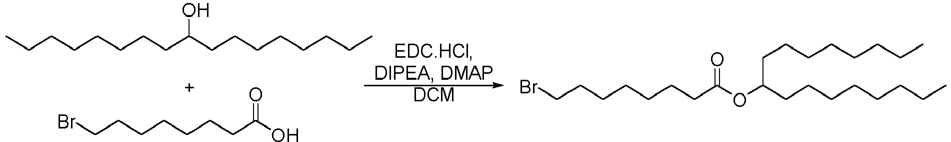

To a 250 mL reaction flask were successively added 8-bromooctanoic acid (22.3 g, 100 mmol), heptadecane-9-ol (25.6 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce 1-octylnonyl 8-bromooctanoate (41.5 g, 90%).

### Example 2: Synthesis of hept-2-ynyl 8-bromooctanoate

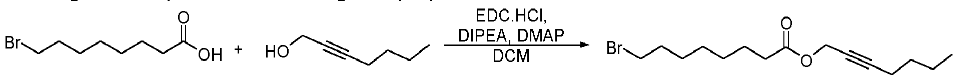

To a 100 mL reaction flask were successively added 8-bromooctanoic acid (2.23 g, 10 mmol), hept-2-yn-1-ol (1.12 g, 10 mmol), and dichloromethane (30mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce hept-2-ynyl 8-bromooctanoate (3.01 g, 95%).

### Example 3: Synthesis of 1-octylnonyl 8-(3-hydroxy-propylamino)octanoate

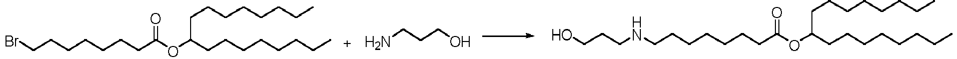

To a 100 mL reaction flask were successively added 1-octylnonyl 8-bromooctanoate (4.61 g, 10 mmol), 3-amino-1-propanol (7.5 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylnonyl 8-(3-hydroxy-propylamino)octanoate (3.32 g, 73%).

### Example 4: Synthesis of Compound 1

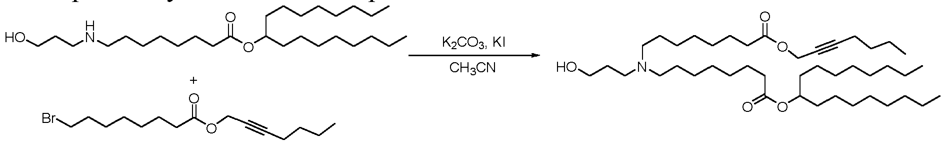

To a 100 mL reaction flask were successively added 1-octylnonyl 8-(3-hydroxy-propylamino)octanoate (455 mg, 1 mmol), hept-2-ynyl 8-bromooctanoate (380 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 1 (560 mg, 81%). ¹H NMR (400 MHz, CDCl₃) δ: ppm 4.88 (m, 1H); 4.65 (m, 2H); 3.68-3.46 (m, 2H); 2.77-2.37 (m, 8H); 2.28 (m, 4H); 1.75-1.42 (m, 14H); 1.39-1.18 (m, 40H); 0.89 (m, 9H). ESI-MS calculated for C₄₃H₈₂NO₅⁺ [M+H]⁺ 692.6, found 692.7.

### Example 5: Synthesis of decan-2-yn-1-yl 8-bromooctanoate

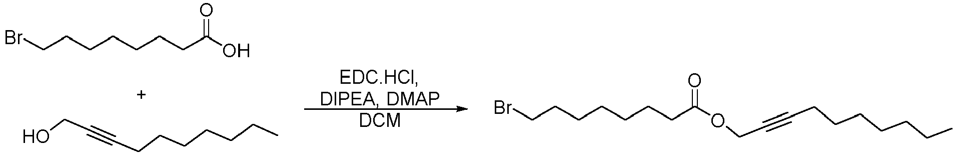

To a 100 mL reaction flask were successively added 8-bromooctanoic acid (2.23 g, 10 mmol), 2-decyn-1-ol (1.54 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce decan-2-yn-1-yl 8-bromooctanoate (3.3 g, 92%).

### Example 6: Synthesis of Compound 4

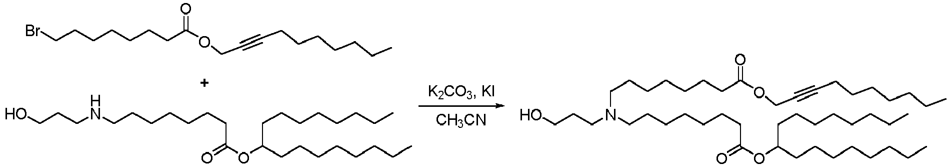

To a 100 mL reaction flask were successively added 1-octylnonyl 8-(3-hydroxy-propylamino)octanoate (455 mg, 1 mmol), decan-2-yn-1-yl 8-bromooctanoate (380 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 4 (558 mg, 76%). ¹H NMR (400 MHz, CDCl₃) δ: ppm 4.87 (m, 1H); 4.68 (m, 2H); 3.64-3.42 (m, 2H); 2.79-2.38 (m, 8H); 2.27 (m, 4H); 1.76-1.43 (m, 14H); 1.41-1.16 (m, 46H); 0.88 (m, 9H). ESI-MS calculated for C₄₆H₈₈NO₅⁺ [M+H]⁺ 734.7, found 734.7.

### Example 7: Synthesis of decan-3-yn-1-yl 8-bromooctanoate

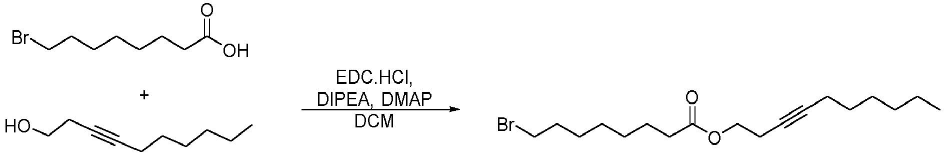

To a 100 mL reaction flask were successively added 8-bromooctanoic acid (2.23 g, 10 mmol), 3-decyn-1-ol (1.54 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce decan-3-yn-1-yl 8-bromooctanoate (3.2 g, 89%).

### Example 8: Synthesis of Compound 7

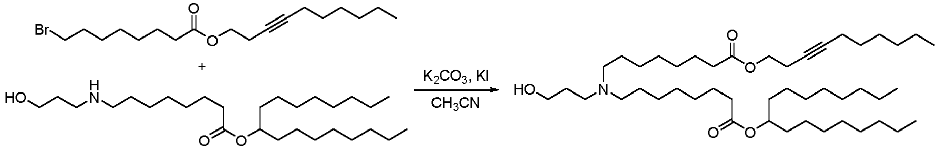

To a 100 mL reaction flask were successively added 1-octylnonyl 8-(3-hydroxy-propylamino)octanoate (455 mg, 1 mmol), decan-3-yn-1-yl 8-bromooctanoate (380 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 7 (558 mg, 76%). ¹H NMR (400 MHz, CDCl₃) δ: ppm 4.89 (m, 1H); 4.67 (m, 2H); 3.66-3.43 (m, 2H); 2.78-2.36 (m, 10H); 2.28 (m, 4H); 1.77-1.45 (m, 14H); 1.40-1.15 (m, 44H); 0.89 (m, 9H). ESI-MS calculated for C₄₆H₈₈NO₅⁺ [M+H]⁺ 734.7, found 734.8.

### Example 9: Synthesis of 1-octylnonyl 8-((2-hydroxyethyl)amino)octanoate

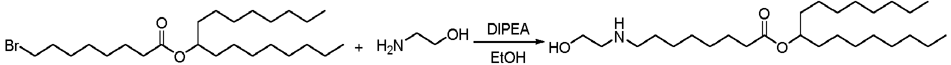

To a 100 mL reaction flask were successively added 1-octylnonyl 8-bromooctanoate (4.61 g, 10 mmol), 2-amino-1-ethanol (6.1 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylnonyl 8-((2-hydroxyethyl)amino)octanoate (3.35 g, 76%).

### Example 10: Synthesis of Compound 14

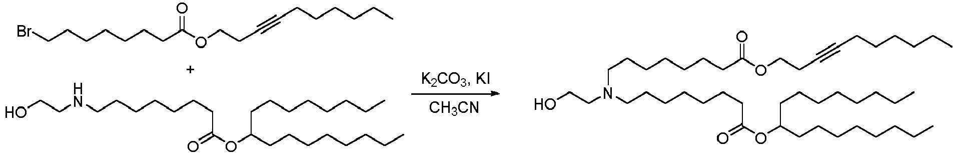

To a 100 mL reaction flask were successively added 1-octylnonyl 8-((2-hydroxyethyl)amino)octanoate (442 mg, 1 mmol), decan-3-yn-1-yl 8-bromooctanoate (380 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 14 (511 mg, 71%). ¹H NMR (400 MHz, CDCl₃) δ: ppm 4.89 (m, 1H); 4.67 (m, 2H); 3.66-3.43 (m, 2H); 2.78-2.36 (m, 10H); 2.28 (m, 4H); 1.77-1.45 (m, 14H); 1.40-1.15 (m, 42H); 0.89 (m, 9H). ESI-MS calculated for C₄₅H₈₆NO₅⁺ [M+H]⁺ 720.7, found 720.8.

### Example 11: Synthesis of 4-nonyn-1-yl 8-bromooctanoate

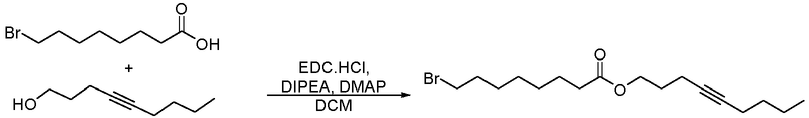

To a 100 mL reaction flask were successively added 8-bromooctanoic acid (2.23 g, 10 mmol), 4-nonyn-1-ol (1.4 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce 4-nonyn-1-yl 8-bromooctanoate (3.0 g, 88%).

### Example 12: Synthesis of 1-octylnonyl

### 8-((4-hydroxylbutyl)amino)octanoate

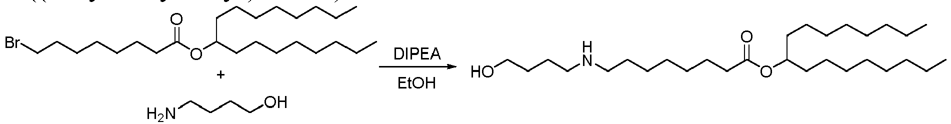

To a 100 mL reaction flask were successively added 1-octylnonyl 8-bromooctanoate (4.61 g, 10 mmol), 4-amino-1-butanol (8.9 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylnonyl 8-((4-hydroxylbutyl)amino)octanoate (3.71 g, 79%).

### Example 13: Synthesis of Compound 17

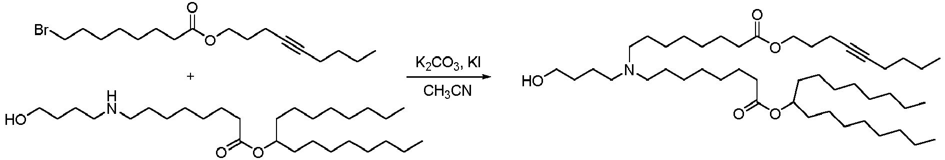

To a 100 mL reaction flask were successively added 1-octylnonyl 8-((4-hydroxylbutyl)amino)octanoate (442 mg, 1 mmol), 4-nonyn-1-yl 8-bromooctanoate (414 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 17 (550 mg, 75%). ¹H NMR (400 MHz, CDCl₃) δ: ppm 4.91 (m, 1H); 4.68 (m, 2H); 3.68-3.44 (m, 2H); 2.79-2.36 (m, 10H); 2.29 (m, 4H); 1.78-1.46 (m, 14H); 1.42-1.16 (m, 44H); 0.89 (m, 9H). ESI-MS calculated for C₄₆H₈₈NO₅⁺ [M+H]⁺ 734.7, found 734.7.

### Example 14: Synthesis of 1-octylnonyl

### 8-((3-hydroxycyclobutyl)amino)octanoate

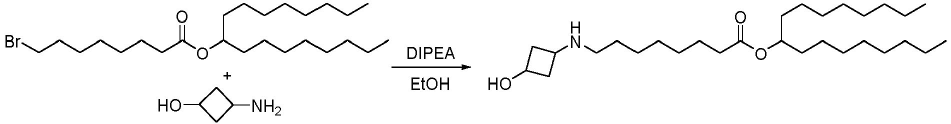

To a 100 mL reaction flask were successively added 1-octylnonyl 8-bromooctanoate (4.61 g, 10 mmol), 3-aminocyclobutanol (8.7 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylnonyl 8-((3-hydroxycyclobutyl)amino)octanoate (3.23 g, 69%).

### Example 15: Synthesis of Compound 18

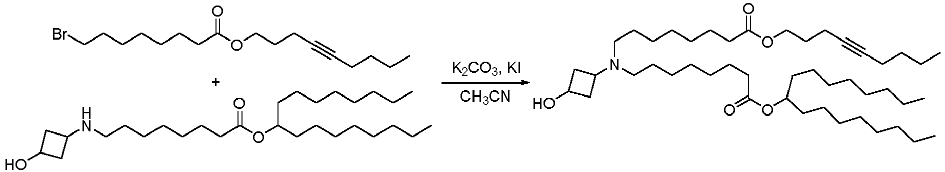

To a 100 mL reaction flask were successively added 1-octylnonyl 8-((3-hydroxycyclobutyl)amino)octanoate (468 mg, 1 mmol), 4-nonyn-1-yl 8-bromooctanoate (414 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 18 (600 mg, 82%). ¹H NMR (400 MHz, CDCl₃) δ: ppm 4.90 (m, 1H); 4.68 (m, 2H); 3.68-3.44 (m, 1H); 2.79-2.36 (m, 10H); 2.29 (m, 3H); 1.78-1.46 (m, 14H); 1.42-1.16 (m, 44H); 0.89 (m, 9H). ESI-MS calculated for C₄₆H₈₆NO₅⁺ [M+H]⁺ 732.7, found 732.7.

### Example 16: Synthesis of 4-nonyn-1-yl 5-bromopentanoate

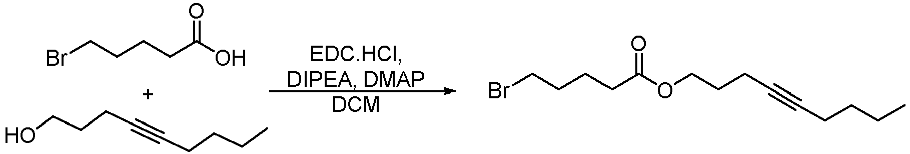

To a 100 mL reaction flask were successively added 5-bromopentanoic acid (1.81 g, 10 mmol), 4-nonyn-1-ol (1.4 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce 4-nonyn-1-yl 5-bromopentanoate (2.7 g, 89%).

### Example 17: Synthesis of Compound 22

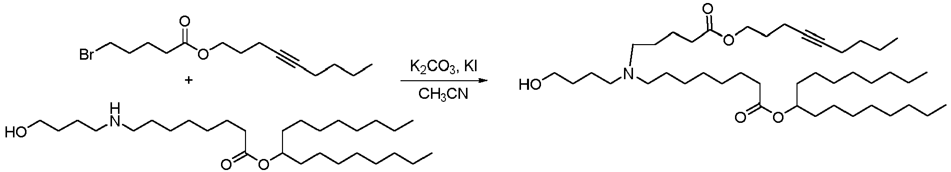

To a 100 mL reaction flask were successively added 1-octylnonyl 8-((4-hydroxylbutyl)amino)octanoate (442 mg, 1 mmol), 4-nonyn-1-yl 5-bromopentanoate (364 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 22 (581 mg, 84%). ¹H NMR (400 MHz, CDCl₃) δ: ppm 4.91 (m, 1H); 4.68 (m, 2H); 3.68-3.44 (m, 2H); 2.79-2.36 (m, 10H); 2.29 (m, 4H); 1.78-1.46 (m, 14H); 1.42-1.16 (m, 38H); 0.89 (m, 9H). ESI-MS calculated for C₄₃H₈₂NO₅⁺ [M+H]⁺ 692.6, found 692.7.

### Example 18: Synthesis of 4-nonyn-1-yl 10-bromodecanoate

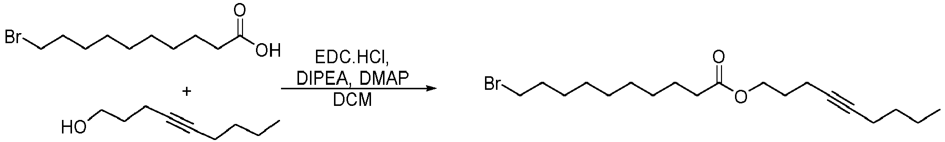

To a 100 mL reaction flask were successively added 10-bromodecanoic acid (2.51 g, 10 mmol), 4-nonyn-1-ol (1.4 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce 4-nonyn-1-yl 10-bromodecanoate (2.95 g, 79%).

### Example 19: Synthesis of Compound 23

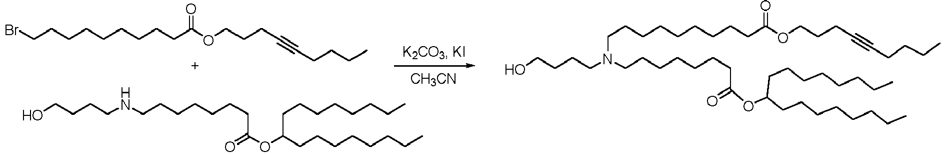

To a 100 mL reaction flask were successively added 1-octylnonyl 8-((2-hydroxylbutyl)amino)octanoate (442 mg, 1 mmol), 4-nonyn-1-yl 10-bromodecanoate (373 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 23 (648 mg, 85%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 48H); 0.88 (m, 9H). ESI-MS calculated for C₄₈H₉₂NO₅⁺ [M+H]⁺ 762.7, found 762.7.

### Example 20: Synthesis of 4-nonyn-1-yl 14-bromotetradecanoate

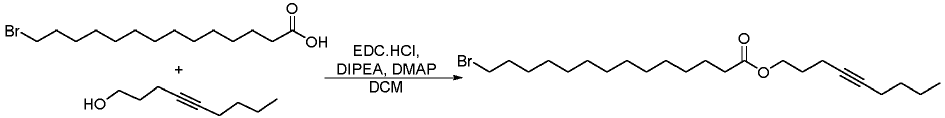

To a 100 mL reaction flask were successively added 14-bromotetradecanoic acid (3.07 g, 10 mmol), 4-nonyn-1-ol (1.4 g, 10 mmol), and dichloromethane (30 mL). The mixture was stirred and dissolved. Then 1-(3-dimethylaminopropyl)-3-ethylcarbodiimide hydrochloride (2.3 g, 12 mmol), 4-dimethylaminopyridine (0.06 g, 0.5 mmol), and N,N-diisopropylethylamine (2.58 g, 20 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 2:1) to produce 4-nonyn-1-yl 14-bromotetradecanoate (3.51 g, 82%).

### Example 21: Synthesis of Compound 24

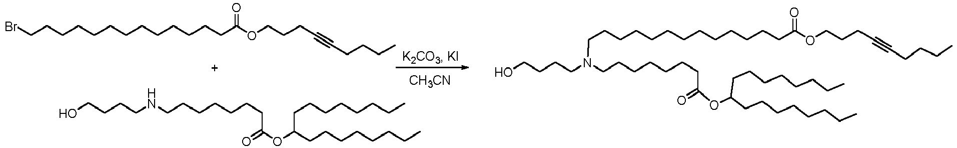

To a 100 mL reaction flask were successively added 1-octylnonyl 8-((4-hydroxylbutyl)amino)octanoate (442 mg, 1 mmol), 4-nonyn-1-yl 14-bromotetradecanoate (429 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 24 (654 mg, 80%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 56H); 0.88 (m, 9H). ESI-MS calculated for C₅₂H₁₀₀NO₅⁺ [M+H]⁺ 818.7, found 818.8.

### Example 22: Synthesis of non-5-yl 8-bromooctanoate

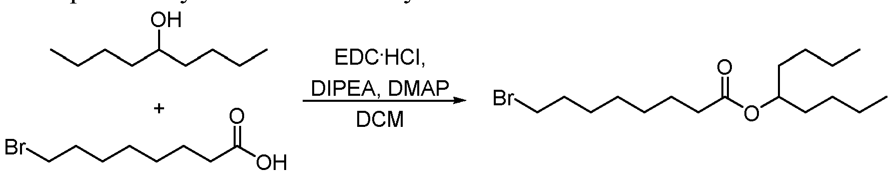

To a 250 mL reaction flask were successively added 8-bromooctanoic acid (22.3 g, 100 mmol), 5-nonanol (14.4 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce non-5-yl 8-bromooctanoate (27.9 g, 82%).

### Example 23: Synthesis of non-5-yl 8-((3-hydroxylpropyl)amino)octanoate

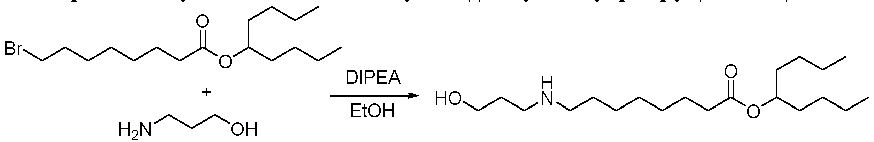

To a 100 mL reaction flask were successively added non-5-yl 8-bromooctanoate (3.49 g, 10 mmol), 3-amino-1-propanol (7.5 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce non-5-yl 8-((3-hydroxylpropyl)amino)octanoate (2.57 g, 75%).

### Example 24: Synthesis of Compound 25

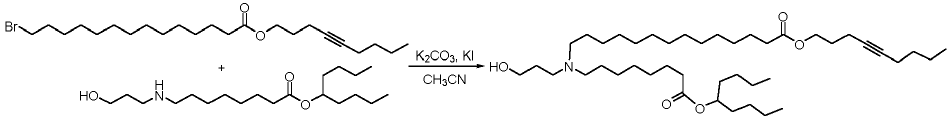

To a 100 mL reaction flask were successively added non-5-yl 8-((3-hydroxylpropyl)amino)octanoate (343 mg, 1 mmol), 4-nonyn-1-yl 14-bromotetradecanoate (429 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 25 (574 mg, 83%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 38H); 0.88 (m, 9H). ESI-MS calculated for C₄₃H₈₂NO₅⁺[M+H]⁺ 692.6, found 692.7.

### Example 25: Synthesis of undecan-5-yl 8-bromooctanoate

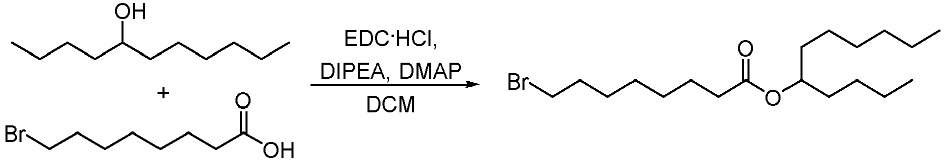

To a 250 mL reaction flask were successively added 8-bromooctanoic acid (22.3 g, 100 mmol), undecane-5-ol (17.2 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce undecane-5-yl 8-bromooctanoate (29.7g, 79%).

### Example 26: Synthesis of 1-butylheptyl

### 8-(3-hydroxy-propylamino)-octanoate

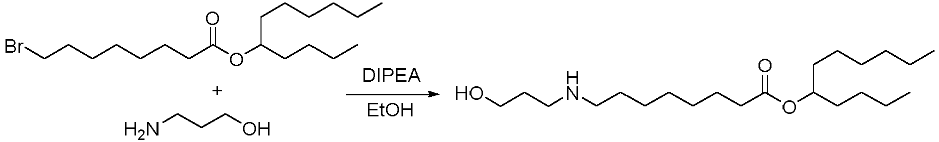

To a 100 mL reaction flask were successively added 1-butylheptyl 8-bromooctanoate (3.49 g, 10 mmol), 3-amino-1-propanol (7.5 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-butylheptyl 8-(3-hydroxy-propylamino)-octanoate (2.93 g, 79%).

### Example 27: Synthesis of Compound 26

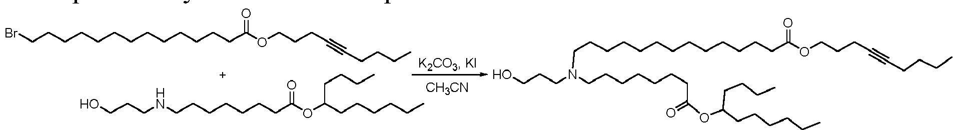

To a 100 mL reaction flask were successively added 1-butylheptyl 8-(3-hydroxy-propylamino)-octanoate (371 mg, 1 mmol), 4-nonyn-1-yl 14-bromotetradecanoate (429 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 26 (583 mg, 81%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 42H); 0.88 (m, 9H). ESI-MS calculated for C₄₅H₈₆NO₅⁺ [M+H]⁺ 720.7, found 720.8.

### Example 28: Synthesis of 1-octylundecyl 8-bromooctanoate

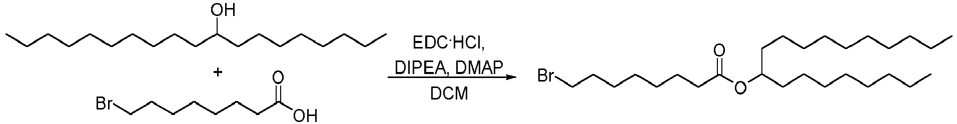

To a 250 mL reaction flask were successively added 8-bromooctanoic acid (22.3 g, 100 mmol), 9-nondecanol (28.4 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce 1-octylundecyl 8-bromooctanoate (38.1 g, 79%).

### Example 29: Synthesis of 1-octylundecyl

### 8-((3-hydroxylpropyl)amino)octanoate

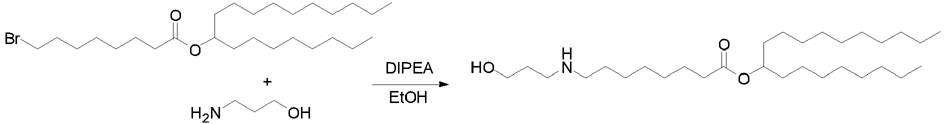

To a 100 mL reaction flask were successively added 1-octylundecyl 8-bromooctanoate (4.89 g, 10 mmol), 3-amino-1-propanol (7.5 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylundecyl 8-((3-hydroxylpropyl)amino)octanoate (3.97 g, 72%).

### Example 30: Synthesis of Compound 27

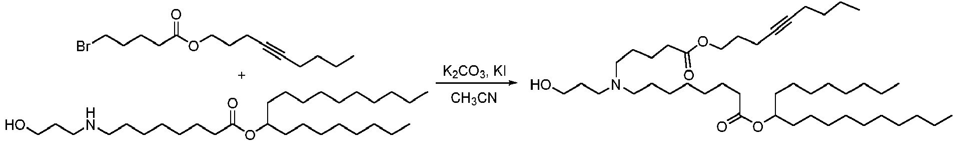

To a 100 mL reaction flask were successively added 1-octylundecyl 8-((3-hydroxylpropyl)amino)octanoate (371 mg, 1 mmol), 4-nonyn-1-yl 5-bromopentanoate (364 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 27 (536 mg, 76%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 40H); 0.88 (m, 9H). ESI-MS calculated for C₄₄H₈₄NO₅⁺[M+H]⁺ 706.6, found 706.6.

### Example 31: Synthesis of 2-hexyldecyl 8-bromooctanoate

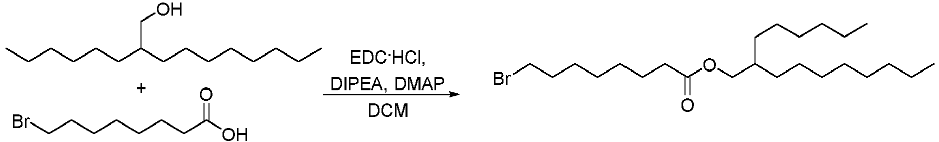

To a 250 mL reaction flask were successively added 8-bromooctanoic acid (22.3 g, 100 mmol), 2-hexyldecan-1-ol (24.2 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce 2-hexyldecyl 8-bromooctanoate (38.1 g, 79%).

### Example 32: Synthesis of 2-hexyldecyl

### 8-((3-hydroxylpropyl)amino)octanoate

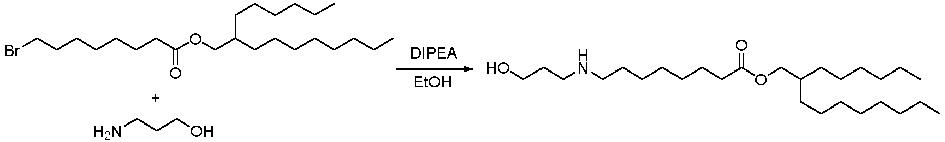

To a 100 mL reaction flask were successively added 2-hexyldecyl 8-bromooctanoate (4.89 g, 10 mmol), 3-amino-1-propanol (7.5 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 2-hexyldecyl 8-((3-hydroxylpropyl)amino)octanoate (3.35 g, 76%).

### Example 33: Synthesis of Compound 28

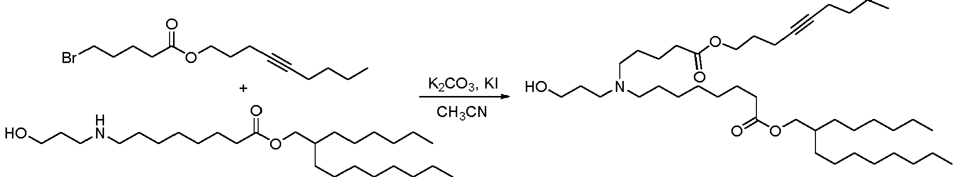

To a 100 mL reaction flask were successively added 2-hexyldecyl 8-((3-hydroxylpropyl)amino)octanoate (441 mg, 1 mmol), 4-nonyn-1-yl 5-bromopentanoate (364 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 28 (471 mg, 71%). ¹H NMR (400 MHz, CDCl₃) δ: ppm 4.90 (m, 1H); 4.67 (m, 2H); 3.69-3.43 (m, 2H); 2.76-2.34 (m, 10H); 2.28 (m, 4H); 1.79-1.47 (m, 14H); 1.44-1.18 (m, 34H); 0.88 (m, 9H). ESI-MS calculated for C₄₁H₇₈NO₅⁺[M+H]⁺ 664.6, found 664.6.

### Example 34: Synthesis of 7-hydroxyheptyl 2-hexyldecanoate

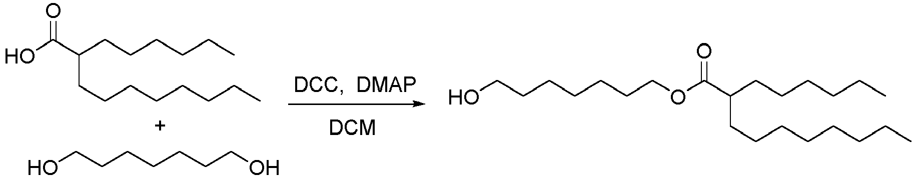

To a 250 mL reaction flask were successively added 2-n-hexyldecanoic acid (25.6 g, 100 mmol), 1,7-heptanediol (66.0 g, 0.5 mol), and dichloromethane (150 mL). The mixture was stirred and dissolved. Then dicyclohexylcarbodiimide (20.6 g, 100 mmol), and 4-dimethylaminopyridine (0.61 g, 5 mmol) were further added. The resulting mixture was reacted at room temperature for 2 hours, washed with water for three times, dried over anhydrous sodium sulfate, concentrated, and then purified with a flash column chromatography system (hexane:ethyl acetate = 5:1 to 1:1) to produce 7-hydroxyheptyl 2-hexyldecanoate (22.6 g, 61%).

### Example 35: Synthesis of heptyl

### 7-((3-hydroxylpropyl)amino)2-hexyldecanoate

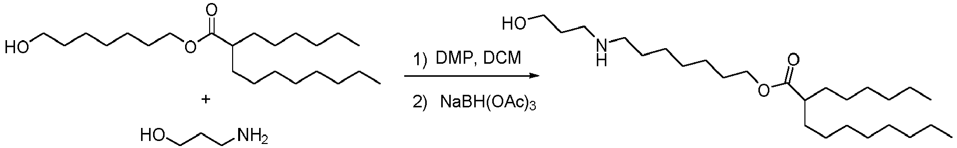

To a 250 mL reaction flask were successively added 7-hydroxyheptyl 2-hexyldecanoate (3.7 g, 10 mmol), and dichloromethane (100 mL), and Dess-Martin periodinane (5.09g, 12 mmol). The mixture was reacted under stirring at room temperature for 12 hours, washed with a saturated sodium bicarbonate solution for three times, washed with water once, and dried over anhydrous sodium sulfate. The desiccating agent was removed by filtering and washed with dichloromethane (10 mL). The organic phases were combined followed by the addition of sodium triacetoxyborohydride (3.18 g, 15 mmol), and then 3-amino-1-propanol (7.5 g, 100 mmol) was added. The resulting mixture was reacted at room temperature for 24 hours, washed with water for three times, dried over anhydrous sodium sulfate, concentrated, and then purified with a flash column chromatography system (dichloromethane:methanol = 20:1 to 5:1) to produce heptyl 7-((3-hydroxylpropyl)amino)2-hexyldecanoate (3.03 g, 71%).

### Example 36: Synthesis of 7-bromoheptyl undec-2-ynoate

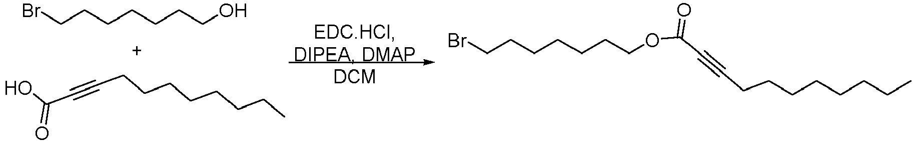

To a 250 mL reaction flask were successively added 8-bromoheptanol (19.5 g, 100 mmol), 2-undecynoic acid (18.2 g, 100 mmol), and dichloromethane (100 mL). The mixture was stirred and dissolved. Then 1-(3-dimethylaminopropyl)-3-ethylcarbodiimide hydrochloride (23.0 g, 120 mmol), 4-dimethylaminopyridine (0.61 g, 5 mmol), and N,N-diisopropylethylamine (25.8 g, 200 mmol) were further added. The resulting mixture was reacted at room temperature for 2 hours, washed with water for three times, dried over anhydrous sodium sulfate, concentrated, and then purified with a flash column chromatography system (hexane: ethyl acetate = 10:1 to 3:1) to produce 7-bromoheptyl undec-2-ynoate (25.4 g, 71%).

### Example 37: Synthesis of Compound 29

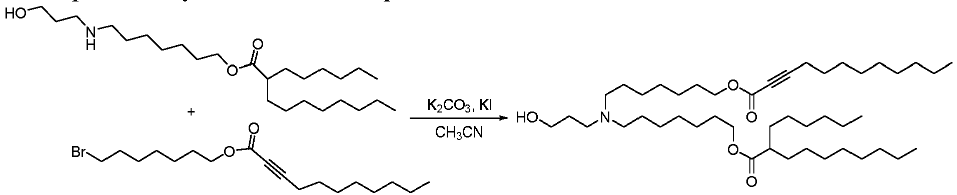

To a 100 mL reaction flask were successively added heptyl 7-((3-hydroxylpropyl)amino)2-hexyldecanoate (427 mg, 1 mmol), 7-bromoheptyl undec-2-ynoate (431 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 29 (454 mg, 63%). ¹H NMR (400 MHz, CDCl₃) δ: ppm 4.88 (m, 4H); 3.67-3.43 (m, 2H); 2.76-2.14 (m, 9H); 1.79-1.47 (m, 12H); 1.44-1.18 (m, 48H); 0.89 (m, 9H). ESI-MS calculated for C₄₅H₈₆NO₅⁺ [M+H]⁺ 720.7, found 720.8.

### Example 38: Synthesis of 1-octylnonyl

### 8-((3-(dimethylamino)propyl)amino)octanoate

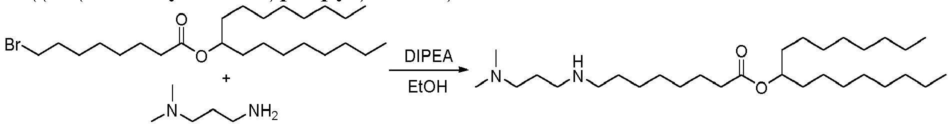

To a 100 mL reaction flask were successively added 1-octylnonyl 8-bromooctanoate (4.61 g, 10 mmol), N,N-dimethylpropylene diamine (8.9 g, 100 mmol), and ethanol (30 mL). The mixture was stirred and dissolved. Then N,N-diisopropylethylamine (2.58 g, 20 mmol) was further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce 1-octylnonyl 8-((3-(dimethylamino)propyl)amino)octanoate (3.62 g, 75%).

### Example 39: Synthesis of Compound 31

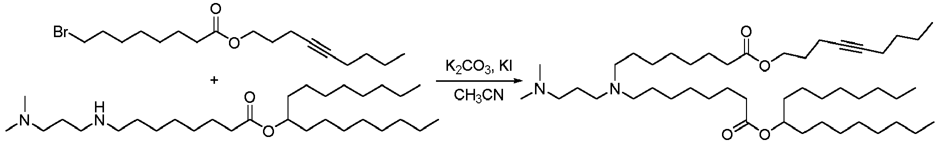

To a 100 mL reaction flask were successively added 1-octylnonyl 8-((3-(dimethylamino)propyl)amino)octanoate (482 mg, 1 mmol), 4-nonyn-1-yl 8-bromooctanoate (414 mg, 1.2 mmol), and acetonitrile (20 mL). The mixture was stirred and dissolved. Then potassium carbonate (276 mg, 2 mmol), and potassium iodide (166 mg, 1 mmol) were further added. The resulting mixture was reacted at room temperature for 24 hours before adding dichloromethane (100 mL), washed with water for three times, dried over anhydrous sodium sulfate, concentrated, and then purified with a flash column chromatography system (dichloromethane:methanol = 20:1 to 5:1) to produce Compound 31 (537 mg, 72%). ¹H NMR (400 MHz, CDCl₃) δ: ppm 4.91 (m, 1H); 4.68 (m, 2H); 2.79-2.36 (m, 20H); 2.29 (m, 4H); 1.78-1.46 (m, 14H); 1.42-1.16 (m, 42H); 0.89 (m, 9H). ESI-MS calculated for C₄₇H₉₁N₂O₄⁺ [M+H]⁺ 747.7, found 747.8.

### Example 40: Evaluation of luciferase mRNA delivery performance in vivo of lipid nanoparticles prepared from amino lipid compounds

Preparation of lipid nanoparticles:
Preparation process I: The amino lipid compound described in the present invention and DOPE, cholesterol, PEG2000-DMG were mixed and dissolved in absolute ethanol in a molar ratio of 45:10:42.5:2.5. Two microinjection pumps were used, and the ratio of ethanol solution to sodium acetate solution (50 mM, pH = 4.0) was controlled to be 1: 3. A crude solution of lipid nanoparticles was prepared in a micro flow channel chip, then dialyzed for 6 hours with 1XPBS at a controlled temperature of 4°C by using a dialysis cassette (Fisher, MWCO 20,000), and filtered with a 0.22 µm microporous filter membrane before use. The mass ratio of the amino lipid compound to luciferase mRNA (Flue mRNA) was about 10:1. The administration route included subcutaneous and intramuscular injections.
Preparation process II: the molar ratio of amino lipid compound to DSPC, cholesterol, PEG2000-DMG was 50:10:38.5:1.5, and the preparation process was identical to the preparation process I. The administration route was tail vein administration.
Animal preparation: 6-week-old male BALB/c mice with body weights of about 20 g were selected, and fed in an SPF-grade feeding room. Animal experiments were strictly carried out according to the guidelines of the national health institution and the requirements of animal ethics.

In vivo delivery: 9 mice were randomly selected per group and injected with lipid nanoparticles at a dose of 0.5 mg/kg by three administration routes: subcutaneous, intramuscular, and tail vein, respectively (three animals per administration route). After 6 hours, 200µL of 10 mg/mL potassium D-fluorescein was injected into each mouse via the tail vein, and after 10 minutes, the mice were placed under an in vivo imaging system (IVIS-200, Xenogen), and the total fluorescence intensity of each mouse was observed and recorded by photographing. The expression intensities of Flue mRNA delivered by the three administration routes of representative amino lipid compounds were shown in Tables 1-3. DLin-MC3 was used as a control. In the in vivo delivery studies of amino lipid compounds without introduction of the alkynyl group (36, 37) and their corresponding amino lipid compounds with introduction of the alkynyl group (17,31), the delivery capacities after introduction of the alkynyl group was significantly enhanced.

**Table 1: Expression intensities of Flue mRNA delivered by the subcutaneous administration of representative amino lipid compounds.**

| No | Structure | Average fluorescence intensity (subcutaneous) |
|---|---|---|
| 1 | 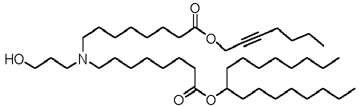 | 1.4E+07 |
| 2 | 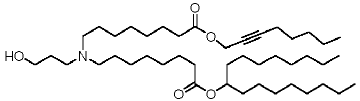 | 1.1E+07 |
| 3 | 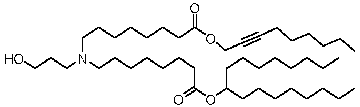 | 2.1E+06 |
| 4 | 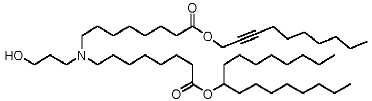 | 2.1E+05 |
| 5 | 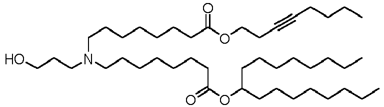 | 7.1E+06 |
| 6 | 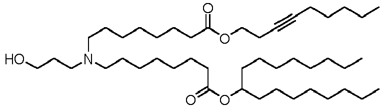 | 2.1E+06 |
| 7 | 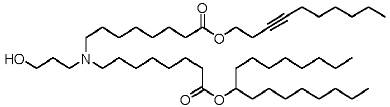 | 2.1E+05 |
| 8 | 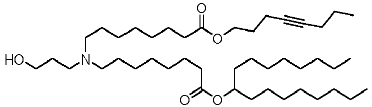 | 7.1E+06 |
| 9 | 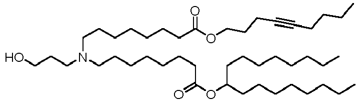 | 2.7E+06 |
| 10 | 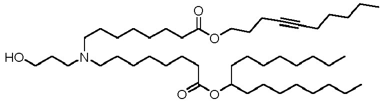 | 3.1E+05 |
| 11 | 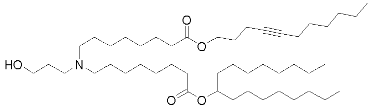 | 2.1E+05 |
| 12 | 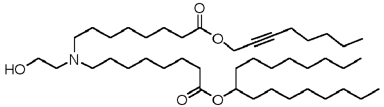 | 3.2E+07 |
| 13 | 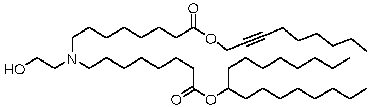 | 2.6E+07 |
| 14 | 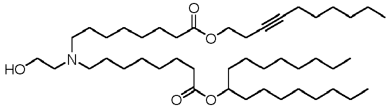 | 2.1E+06 |
| 15 | 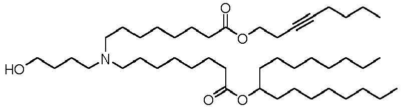 | 3.1E+05 |
| 16 | 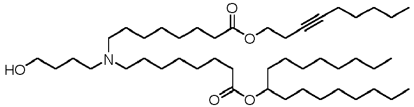 | 2.7E+06 |
| 17 | 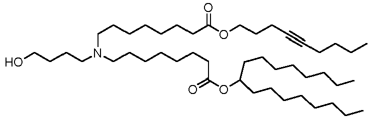 | 2.5E+06 |
| 20 | 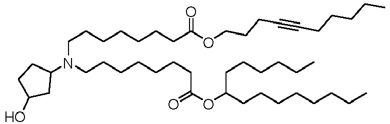 | 3.7E+07 |
| 21 | 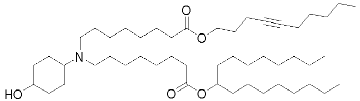 | 4.1E+06 |
| 22 | 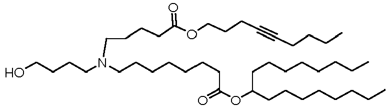 | 2.8E+07 |
| 25 | 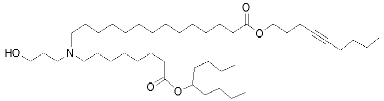 | 2.6E+07 |
| 26 | 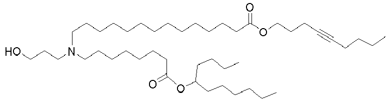 | 1.7E+06 |
| 28 | 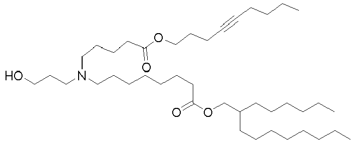 | 2.1E+06 |
| 29 | 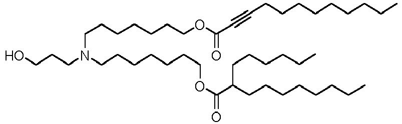 | 8.1E+06 |
| 30 | 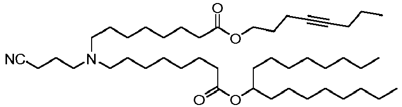 | 7.1E+06 |
| 31 | 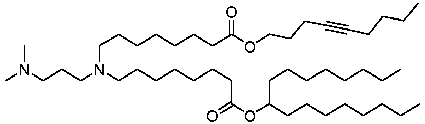 | 5.1E+05 |
| 32 | 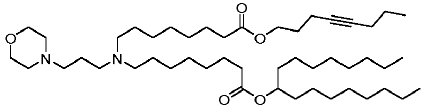 | 7.1E+05 |
| 33 | 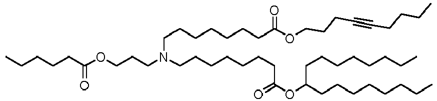 | 3.1E+06 |
| 34 | 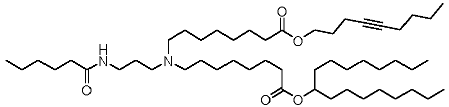 | 5.3E+05 |
| 35 | DLin-MC3 | 4.1E+04 |
| 36 | 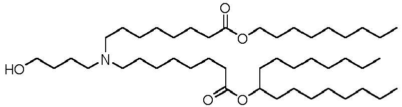 | 1.1E+05 |
| 37 | 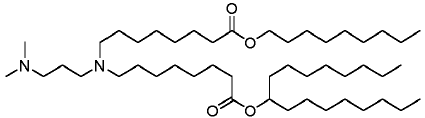 | 2.0E+05 |

**Table 2: Expression intensities of Flue mRNA delivered by the intramuscular administration of representative amino lipid compounds.**

| No. | Structure | Average fluorescence intensity (intramuscular) |
|---|---|---|
| 2 | 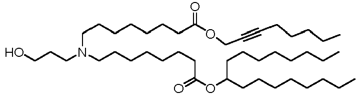 | 2.4E+06 |
| 3 | 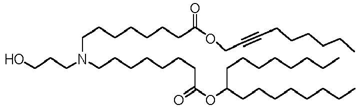 | 2.1E+06 |
| 4 | 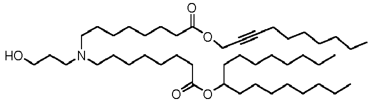 | 2.1E+06 |
| 6 | 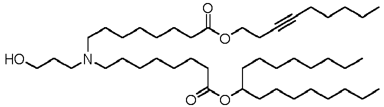 | 2.1E+05 |
| 7 | 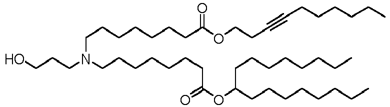 | 4.1E+05 |
| 8 | 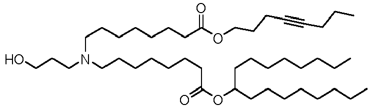 | 2.1E+07 |
| 9 | 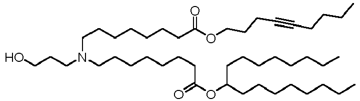 | 5.1E+06 |
| 10 | 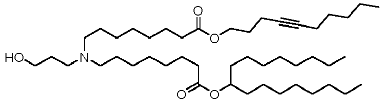 | 2.1E+05 |
| 11 | 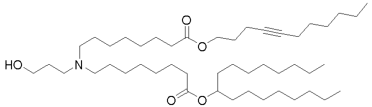 | 2.1E+06 |
| 12 | 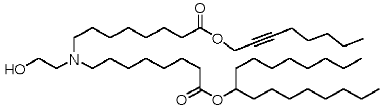 | 2.1E+06 |
| 13 | 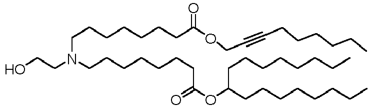 | 2.1E+06 |
| 14 | 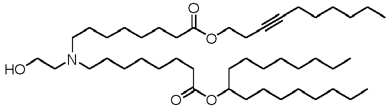 | 4.1E+06 |
| 15 | 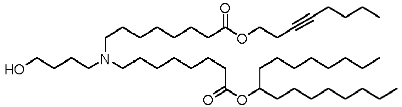 | 2.8E+07 |
| 16 | 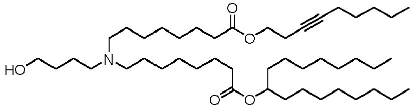 | 1.4E+07 |
| 17 | 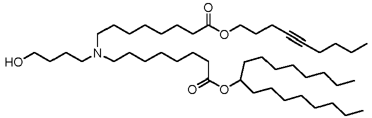 | 2.7E+06 |
| 19 | 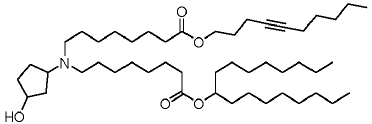 | 2.1E+05 |
| 20 | 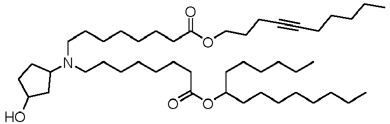 | 5.2E+05 |
| 21 | 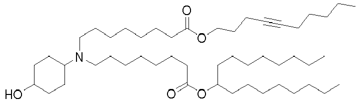 | 1.1E+07 |
| 22 | 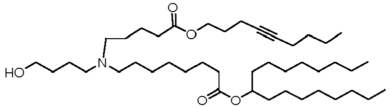 | 3.1E+05 |
| 23 | 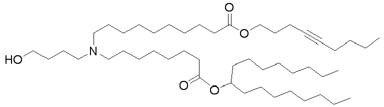 | 2.7E+05 |
| 24 | 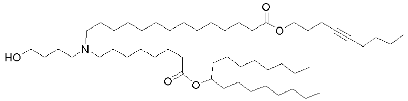 | 2.1E+05 |
| 25 | 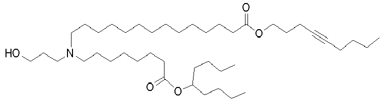 | 2.1E+05 |
| 26 | 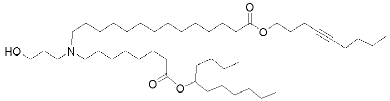 | 6.1E+07 |
| 27 | 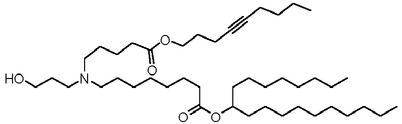 | 2.1E+07 |
| 28 | 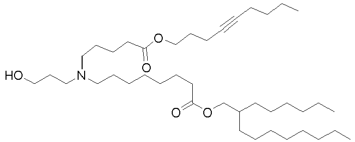 | 6.1E+07 |
| 29 | 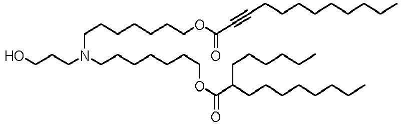 | 1.8E+07 |
| 30 | 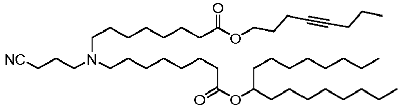 | 1.1E+07 |
| 31 | 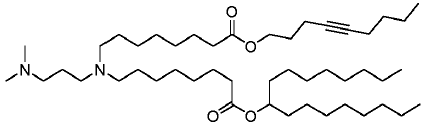 | 1.5E+07 |
| 32 | 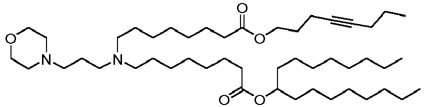 | 2.8E+07 |
| 33 | 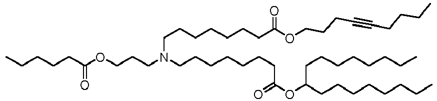 | 1.1E+07 |
| 34 | 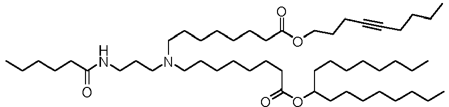 | 4.8E+06 |
| 35 | DLin-MC3 | 3.9E+03 |
| 37 | 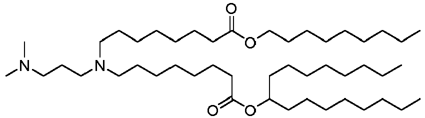 | 2.5E+06 |

**Table 3: Expression intensities of Flue mRNA delivered by the tail vein administration of representative amino lipid compounds.**

| No. | Structure | Average fluorescence intensity (tail vein) |
|---|---|---|
| 3 | 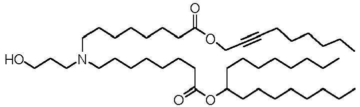 | 2.1E+07 |
| 4 | 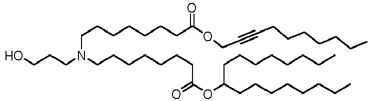 | 2.9E+07 |
| 7 | 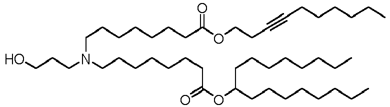 | 8.1E+06 |
| 8 | 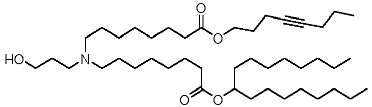 | 6.1E+06 |
| 9 | 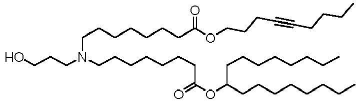 | 2.8E+07 |
| 10 | 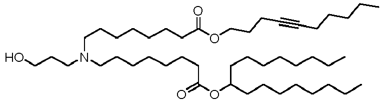 | 2.5E+07 |
| 11 | 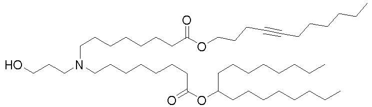 | 5.1E+07 |
| 14 | 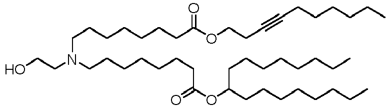 | 1.1E+07 |
| 17 | 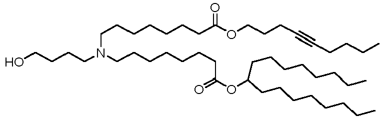 | 5.1E+07 |
| 18 | 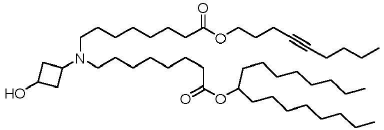 | 7.1E+07 |
| 19 | 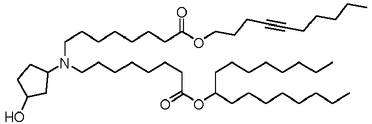 | 9.1E+06 |
| 23 | 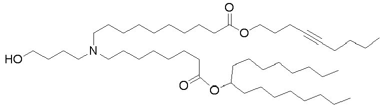 | 6.1E+07 |
| 24 | 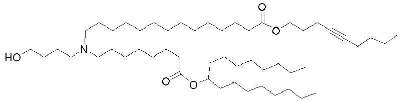 | 7.8E+06 |
| 35 | DLin-MC3 | 6.2E+06 |
| 36 | 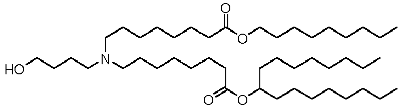 | 4.2E+05 |

### Abbreviation list

DNA: Deoxyribonucleic acid
RNA: ribonucleic acid
DOPE: Dioleoylphosphatidylethanolamine
DSPC: Distearoylphosphatidylcholine
PEG2000-DMG: 1-(Monomethoxypolyethylene glycol)-2, 3 dimyristoyl glycerol
KD: kilodalton
PBS: Phosphate buffer solution
DLin-MC3: (6Z,9Z,28Z,31Z)-heptatriacont-6,9,28,31-tetraene-19-yl 4-(N,N-dimethylamino)butanoate.

## Claims

1. A compound of the following formula I: 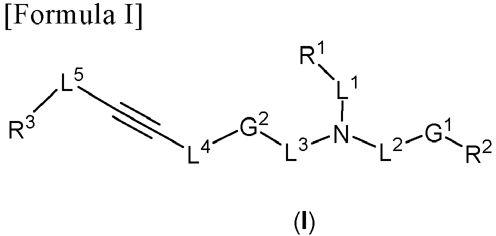 or a pharmaceutically acceptable salt, prodrug or stereoisomer thereof, wherein:
G¹ and G² are identical or different and each independently selected from -O(C=O)-, -(C=O)O-, -C(=O)-, -O-, -S(O)ₚ-, -S-S-, -C(=O)S-, -SC(=O)-, -NR^{a}C(=O)-, -C(=O)NR^{a}-, -NR^{a}C(=O)NR^{a}-, -OC(=O)NR^{a}- or -NR^{a}C(=O)O-; wherein p=0, 1, or 2; R^{a} is H or C₁-C₁₂ hydrocarbyl;
L¹, L², L³, L⁴ and L⁵ are identical to or different from each other, and each independently selected from absence, C₁-C₂₄alkylene, C₂-C₂₄alkenylene, C₃-C₈cycloalkylene, C₃-C₈cycloalkenylene, or optionally substituted 4- to 10-membered heterocycle containing heteroatom(s) selected from nitrogen, sulphur, and oxygen, wherein the C₁-C₂₄alkylene, the C₂-C₂₄alkenylene, the C₃-C₈cycloalkylene, and the C₃-C₈cycloalkenylene are optionally substituted by one or more substituent groups selected from hydrocarbyl, carboxyl, acyl, and alkoxy;
R² is a branched C₆-C₂₄alkyl or a branched C₆-C₂₄alkenyl;
R¹ and R³ are identical or different and each independently selected from H, OR^{1a}, CN, -C(=O)OR^{1a}, -OC(=O)R^{1a}, -NR^{1b}C(=O)R^{1a} or -NR^{1a}R^{1b}; wherein
each of R^{1a} and R^{1b} is H or C₁-C₁₂ hydrocarbyl.

2. The compound according to claim 1, which has the following structure: 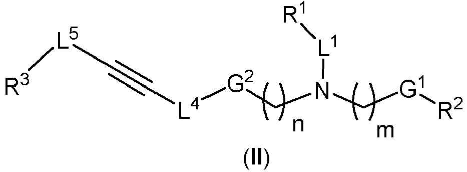 wherein m and n, identical or different, each independently is an integral number from 1 to 13.

3. The compound according to claim 1, which has the following structure: 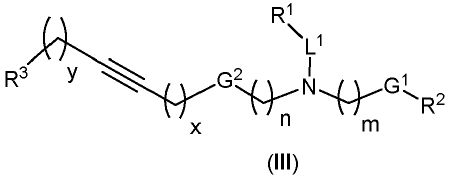 wherein x, y, m, and n, identical or different, each independently is an integral number from 0 to 13.

4. The compound according to claim 1, which has one of the following structures (IVa) and (IVb): 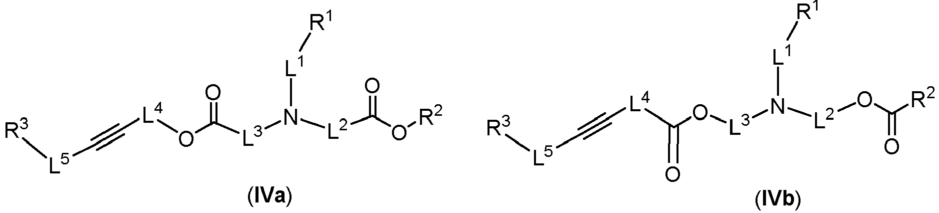 L¹, L², L³, L⁴, and L⁵ are identical or different and each independently selected from C₁-C₁₂alkylene, C₂-C₁₂alkenylene, C₃-C₈cycloalkylene, and C₃-C₈cycloalkenylene.

5. The compound according to claim 4, which has one of the following structures (Va) and (Vb): 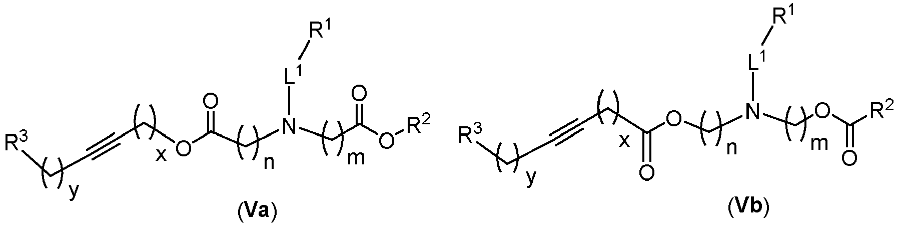 wherein x, y, m, and n, identical or different, each independently is an integral number from 1 to 12.

6. The compound according to any one of claims 1-5, **characterized in that** R³ is H.

7. The compound according to any one of claims 1-5, **characterized in that** R² is selected from one of the following structures:

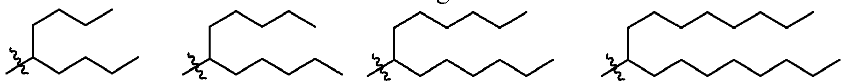

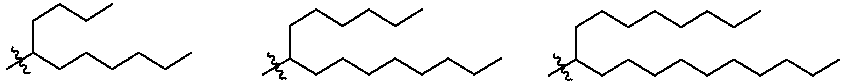

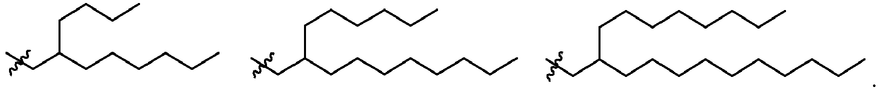

8. The compound according to claim 1, **characterized in that**
G¹ and G² are identical or different and each independently selected from -O(C=O)- and -(C=O)O-;
L¹, L², L³, L⁴ and L⁵ are identical to or different from each other, and each independently is absent or represents C₁-C₁₃alkylene or C₄-C₆cycloalkyl; R¹ and R³ are identical or different and each independently selected from H, OR^{1a}, CN, 4-6 membered saturated heterocyclyl containing one or two heteroatoms selected from N and O, -OC(=O)R^{1a}, -NR^{1b}C(=O)R^{1a} or -NR^{1a}R^{1b}; wherein each of R^{1a} and R^{1b} is H or C₁-C₁₂alkyl;
R² is a branched C₆-C₂₄alkyl.

9. The compound according to claim 1, **characterized in that**
G¹ and G² are identical or different and each independently selected from -O(C=O)- , -(C=O)O-;
L¹ is C₁-C₁₃alkylene or C₄-C₆ cycloalkyl;
L², L³, L⁴ and L⁵ are identical to or different from each other, and each independently is absent or represents C₁-C₁₃alkylene;
R¹ is selected from OR^{1a}, CN, 4-6 membered saturated heterocyclyl containing one or two heteroatoms selected from N and O, -OC(=O)R^{1a}, -NR^{1b}C(=O)R^{1a} or -NR^{1a}R^{1b}; wherein each of R^{1a} and R^{1b} is H or C1-C₆ alkyl;
R² is a branched C₆-C₂₄alkyl;
R³ is C1-C₆ alkyl.

10. The compound according to claim 1, **characterized in that**
G¹ and G² are identical or different and each is independently selected from -O(C=O)- and -(C=O)O-;
L¹ is C₁-C₆ alkylene or C₄-C₆ cycloalkyl;
L² is heptylene;
L³ is C₄-C₁₃alkylene;
L⁴ is absent or represents C₁-C₃alkylene;
L⁵ is C₂-C₈alkylene;
R¹ is selected from OH, CN, morpholinyl, -OC(=O)(CH₂)₄CH₃, -NHC(=O)(CH₂)₄CH₃ and -N(CH₃)₂;
R² is selected from 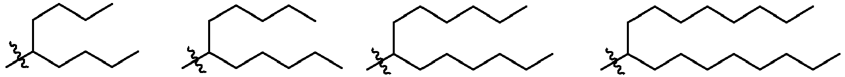 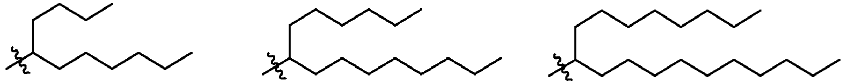 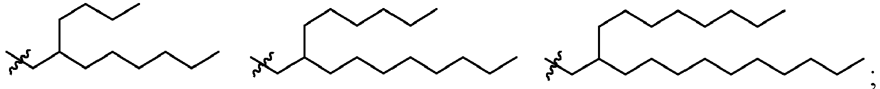
R³ is methyl.

11. The compound according to claim 1, which is selected from the following compounds:
| No. | Structure |
|---|---|
| 1 | 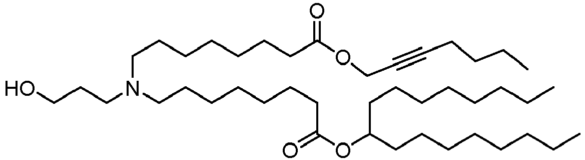 |
| 2 | 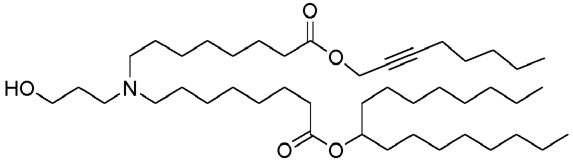 |
| 3 | 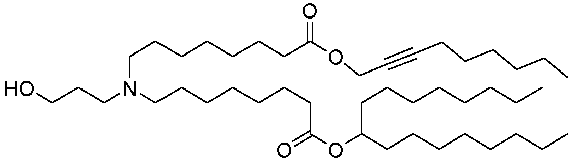 |
| 4 | 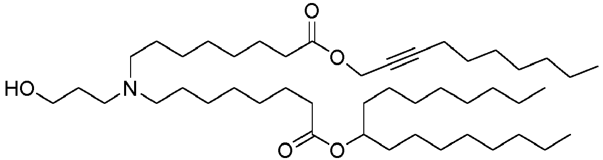 |
| 5 | 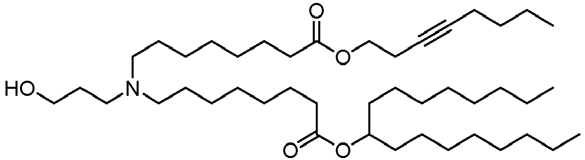 |
| 6 | 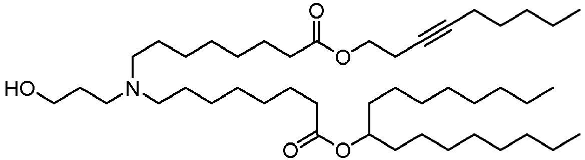 |
| 7 | 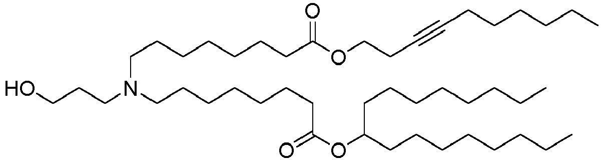 |
| 8 | 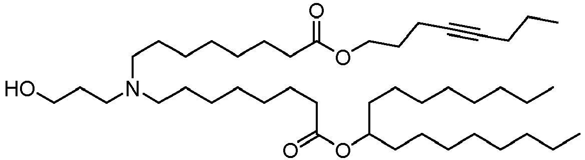 |
| 9 | 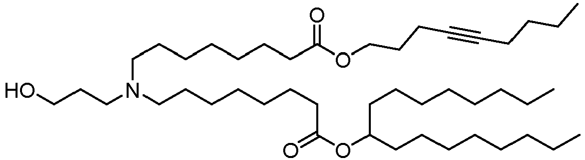 |
| 10 | 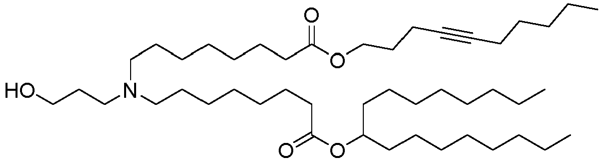 |
| 11 | 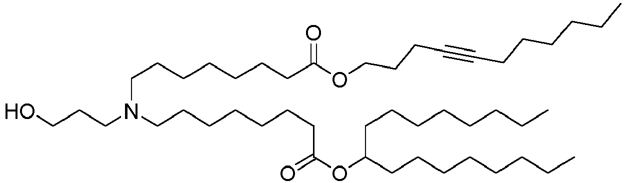 |
| 12 | 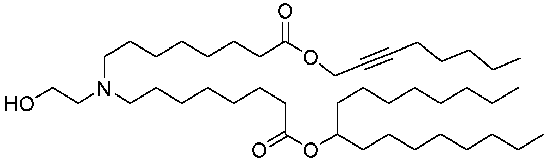 |
| 13 | 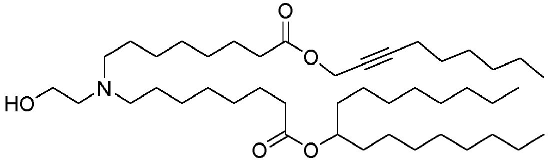 |
| 14 | 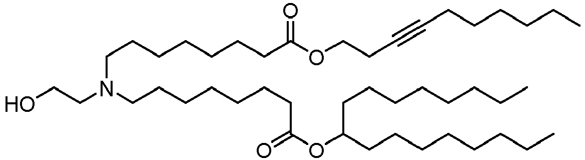 |
| 15 | 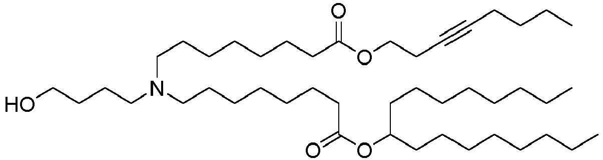 |
| 16 | 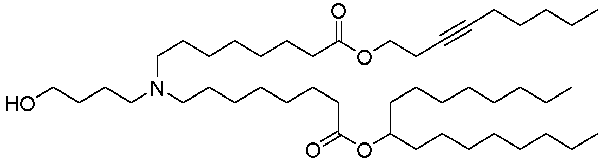 |
| 17 | 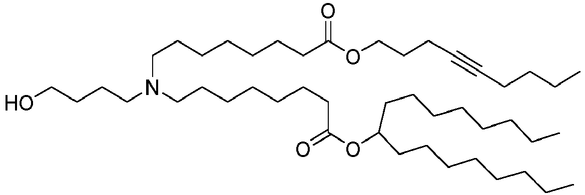 |
| 18 | 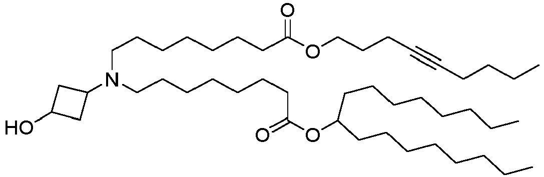 |
| 19 | 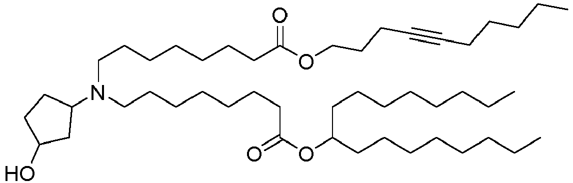 |
| 20 | 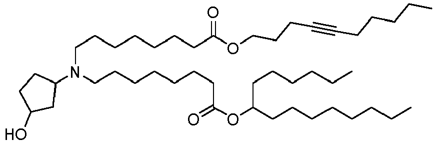 |
| 21 | 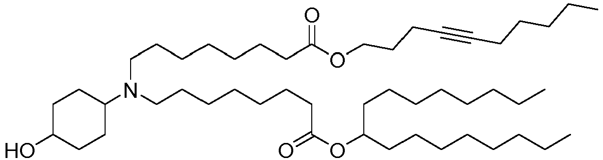 |
| 22 | 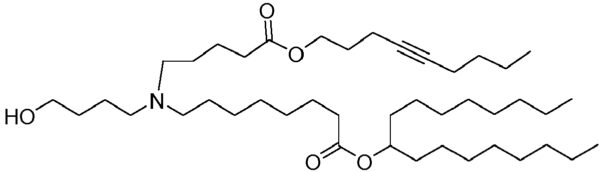 |
| 23 | 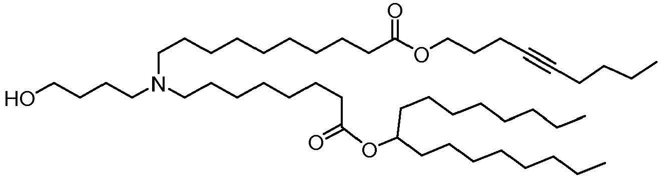 |
| 24 | 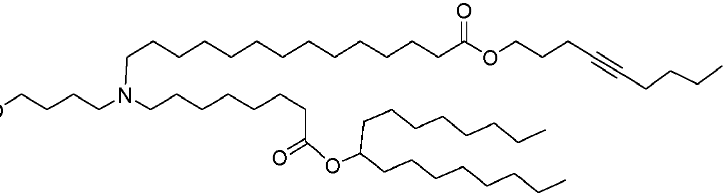 |
| 25 | 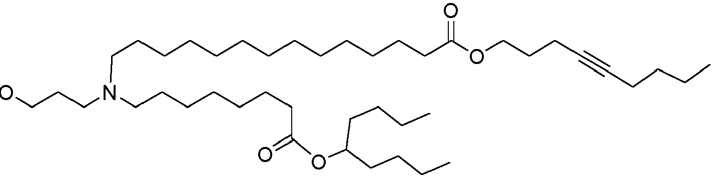 |
| 26 | 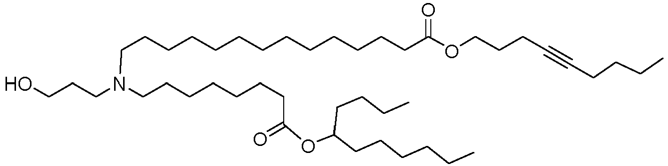 |
| 27 | 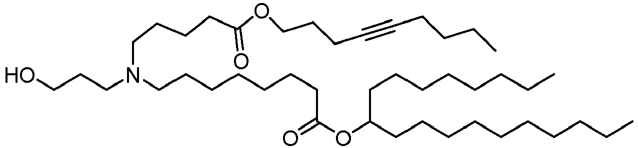 |
| 28 | 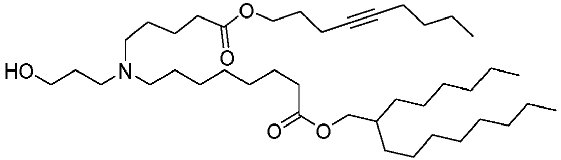 |
| 29 | 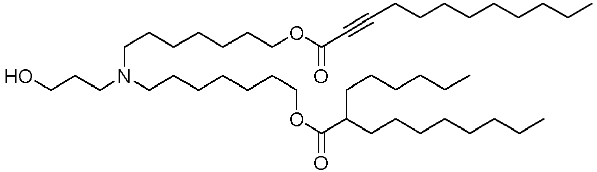 |
| 30 | 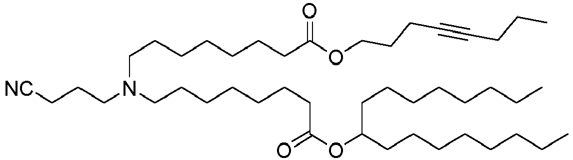 |
| 31 | 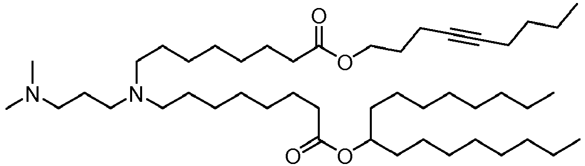 |
| 32 | 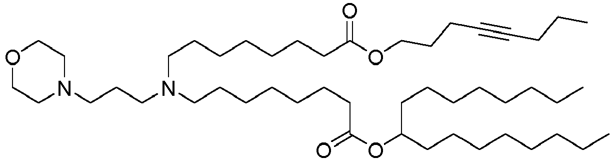 |
| 33 | 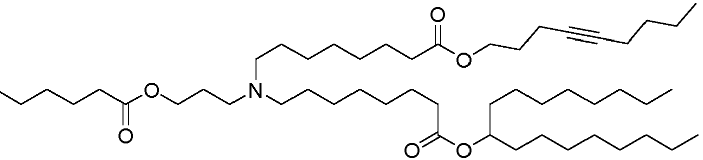 |
| 34 | 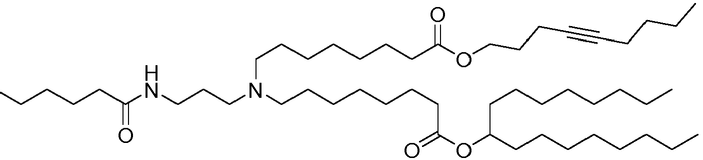 |

12. A lipid nanoparticle, **characterized in that** the lipid nanoparticle contains the compound according to any one of claims 1-11.

13. The lipid nanoparticle according to claim 12, wherein the lipid nanoparticle further contains one or more of helper lipid, sterol, polyethylene glycol lipid and bioactive agent;
preferably, the helper lipid is non-cationic lipid, more preferably the helper lipid is non-cationic phospholipid, further preferably, the non-cationic lipid is DOPE or DSPC;
preferably, the sterol is cholesterol;
preferably, the polyethylene glycol lipid is PEG2000-DMG;
preferably, the bioactive agent is one or more of nucleic acid, antineoplastic agent, antibiotic, immunomodulator, anti-inflammatory agent, agent acting on the central nervous system, antigen or fragment thereof, peptide, protein, antibody, vaccine and small molecule; more preferably, the nucleic acid is RNA, messenger RNA (mRNA), antisense oligonucleotide, DNA, plasmid, ribosomal RNA (rRNA), micro RNA (miRNA), transfer RNA (tRNA), small inhibitory RNA (siRNA) and small nuclear RNA (snRNA).

14. A process for preparing the compound according to any one of claims 1-11, comprising the following steps:
(1) compound represented by X-L²-G¹-R² is reacted with a compound represented by R¹-L¹-NH₂ in the presence of an organic base to produce a compound represented by R¹-L¹-NH-L²-G¹-R², wherein X is halogen; or,
a compound represented by HO-L²-G¹-R² undergoes oxidation reaction in the presence of Dess-Martin periodinane, then the resulting product is reacted with R¹-L¹-NH₂ in the presence borohydride to produce a compound represented by R¹-L¹-NH-L²-G¹-R²;
(2) the resulting compound represented by R¹-L¹-NH-L²-G¹-R² is reacted with 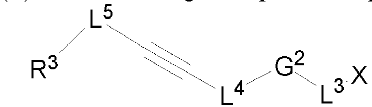 to produce the compound according to any one of claims 3-10, wherein X is halogen;
wherein G¹, G², L¹, L², L³, L⁴, L⁵, R¹, R² and R³ are defined as those in any one of claims 1-11.

15. Use of the compound according to any one of claims 1-11 and the lipid nanoparticle according to claim 12 or 13 in the manufacture of a medicament, wherein the medicament is a medicament for use in gene therapy, gene vaccination, antisense therapy or therapy by interfering RNA;
preferably, the gene therapy is useful for the treatment of cancer and genetic disease; more preferably, the cancer is one or more of lung cancer, stomach cancer, liver cancer, esophagus cancer, colon cancer, pancreatic cancer, brain cancer, lymphatic cancer, leukaemia and prostatic cancer, the genetic disease is one or more of hemophilia, Mediterranean anemia, and Gaucher's disease;
preferably, the gene vaccination is used in the treatment of cancer, allergy, toxicity and infection by pathogens; more preferably, the pathogen is one or more of virus, bacteria and fungi.

16. Use of the compound according to any one of claims 1-11 and the lipid nanoparticle according to claim 12 or 13 in the manufacture of a medicament for nucleic acid transfer, preferably, the nucleic acid is RNA, messenger RNA (mRNA), antisense oligonucleotide, DNA, plasmid, ribosomal RNA (rRNA), micro RNA (miRNA), transfer RNA (tRNA), small inhibitory RNA (siRNA) and small nuclear RNA (snRNA).
